(19) **Europäisches Patentamt / European Patent Office / Office européen des brevets**

(11) **EP 4 339 209 A1**

(12) # EUROPEAN PATENT APPLICATION
published in accordance with Art. 153(4) EPC

(43) Date of publication:
**20.03.2024 Bulletin 2024/12**

(21) Application number: **22807809.3**

(22) Date of filing: **10.05.2022**

(51) International Patent Classification (IPC):
**C07K 16/28** $^{(2006.01)}$    **A61P 35/00** $^{(2006.01)}$
**A61K 39/00** $^{(2006.01)}$

(52) Cooperative Patent Classification (CPC):
**A61K 39/00; A61P 35/00; C07K 16/28**

(86) International application number:
**PCT/KR2022/006697**

(87) International publication number:
**WO 2022/240161 (17.11.2022 Gazette 2022/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.05.2021 KR 20210060012**

(71) Applicant: **MediMabbio Inc.
Seongnam-si, Gyeonggi-do 13449 (KR)**

(72) Inventors:
• **KANG, Yuhoi
  Seongnam-si, Gyeonggi-do 13449 (KR)**
• **JO, Hongseok
  Seongnam-si, Gyeonggi-do 13449 (KR)**

(74) Representative: **Gevers Patents
Intellectual Property House
Holidaystraat 5
1831 Diegem (BE)**

Remarks:
The complete document including Reference Table(s) and the Sequence Listing(s) can be downloaded from the EPO website

(54) **ANTI-GITR ANTIBODIES AND USES THEREOF**

(57)    The present invention relates to novel anti-GITR antibodies and uses thereof for immunity enhancement, cancer prevention, and prevention and/or treatment of immune diseases.

【Fig. 14】

Mean Tumor Volume ± SEM

# EP 4 339 209 A1

**Description**

**Technical Field**

<u>CROSS-REFERENCE TO RELATED APPLICATION(S)</u>

This application claims the benefits of KR 10-2021-0060012 filed on May 10, 2021 with the Korean Intellectual Property Office, the entire disclosure of which is herein incorporated by reference.

Disclosed are a novel anti-GITR antibody and uses thereof for immunopotentiation and for preventing and/or treating cancer and immune-related diseases.

**Background Art**

Cancer immunotherapy is a method of treating cancer using the body's immune response, and is referred to as the fourth treatment for cancer, following chemotherapy, surgical therapy, and radiotherapy. Cancer immunotherapy requires a mechanism that activates the immune system to increase recognition and response to tumor cells. Activation of the immune system involves a complex mechanism that includes the function of various cells such as antigen-presenting cells, which are crucial for initiating antigen-specific responses, and effector cells responsible for the destruction of tumor cells.

Glucocorticoid-induced TNFR-related protein (GITR) is a member of the tumor necrosis factor receptor (TNFR) superfamily and functions to activate T cells to promote immune responses.

Therefore, there is a need for developing a drug that exhibits an immunopotentiating effect by activating GITR.

**Disclosure**

**Technical Problem**

Provided are an anti-GITR antibody binding to GITR and a pharmaceutical use thereof.

The anti-GITR antibody or antigen-binding fragment thereof provided herein may function as an agonist for GITR.

The anti-GITR antibody or antigen-binding fragment thereof may include:

a heavy chain complementarity determining region (CDR) composed of a polypeptide including the amino acid sequence of SEQ ID NO: 1 (CDR-H1), a polypeptide including the amino acid sequence of SEQ ID NO: 2 (CDR-H2), and a polypeptide including the amino acid sequence of SEQ ID NO: 3 (CDR-H3), or a heavy chain variable region including the heavy chain complementarity determining region; and
a light chain complementarity determining region (CDR) composed of a polypeptide including the amino acid sequence of SEQ ID NO: 4 (CDR-L1), a polypeptide including the amino acid sequence of SEQ ID NO: 5 (CDR-L2), and a polypeptide including the amino acid sequence of SEQ ID NO: 6 (CDR-L3), or a light chain variable region including the light chain complementarity determining region

In an embodiment, the polypeptide including the amino acid sequence of SEQ ID NO: 1 (CDR-H1) may include the amino acid sequence of SEQ ID NO: 7, 8, or 9 and the polypeptide including the amino acid sequence of SEQ ID NO: 5 (CDR-L2) may include the amino acid sequence of SEQ ID NO: 10, 11, or 12.

In an embodiment, the anti-GITR antibody or the antigen-binding fragment thereof may include:

a heavy variable region including the amino acid sequence of SEQ ID NO: 13, 14, 15, 16, 17, or 18; and
a light chain variable region including the amino acid sequence of SEQ ID NO: 19, 20, 21, 22, 23, or 24.

Another aspect provides an immunopotentiator or an immunopotent pharmaceutical composition, each including the anti-GITR antibody or antigen-binding fragment thereof as an active ingredient.

Another aspect provides a pharmaceutical composition including the anti-GITR antibody or antigen-binding fragment thereof as an active ingredient for prevention and/or treatment of an immune-related disease.

Another aspect provides an anticancer agent or a pharmaceutical composition for prevention and/or treatment of cancer, each including the anti-GITR antibody or antigen-binding fragment thereof as an active ingredient.

Another aspect provides an immunopotentiating method including a step of administering a pharmaceutically effective amount of the anti-GITR antibody or antigen-binding fragment thereof to a subject in need of immunopotentiation.

Another aspect provides a method for preventing and/or treating an immune-related disease, the method including a step of administering a pharmaceutically effective amount of the anti-GITR antibody or antigen-binding fragment thereof to a subject in need of immune-related disease prevention or treatment.

Another aspect provides a method for preventing and/or treating cancer, the method including a step of administering a pharmaceutically effective amount of the anti-GITR antibody or antigen-binding fragment thereof to a subject in need of cancer prevention and/or treatment.

Another aspect provides a use of the anti-GITR antibody or antigen-binding fragment thereof for immunopotentiation or for preparing an immunopotentiator.

Another aspect provides a use of the anti-GITR antibody or antigen-binding fragment thereof for preventing and/or treating an immune-related disease or for preparing a drug for prevention and/or treatment of an immune-related disease. Another aspect provides a use of the anti-GITR antibody or antigen-binding fragment thereof for preventing and/or treating cancer or for preparing a drug for prevention and/or treatment of cancer.

Another aspect provides a nucleic acid molecule encoding the heavy chain complementarity determining region, heavy chain variable region, or heavy chain of the anti-GITR antibody.

Another aspect provides a nucleic acid molecule encoding the light chain complementarity determining region, light chain variable region, or light chain of the anti-GITR antibody.

Another aspect provides a recombinant vector carrying a nucleic acid molecule coding for the heavy chain complementarity determining region, heavy chain variable region, or heavy chain of the anti-GITR antibody and a nucleic acid molecule coding for the light chain complementarity determining region, light chain variable region, or light chain of the anti-GITR antibody in combination, or separate recombinant vectors carrying a nucleic acid molecule coding for the heavy chain complementarity determining region, heavy chain variable region, or heavy chain of the anti-GITR antibody and a nucleic acid molecule coding for the light chain complementarity determining region, light chain variable region, or light chain of the anti-GITR antibody, respectively. The recombinant vectors may be expression vectors for expressing the nucleic acid molecules.

Another aspect provides a recombinant cell anchoring the recombinant vector thereat.

Another aspect provides a method for producing the anti-GITR antibody or antigen-binding fragment thereof, the method including a step of expressing the nucleic acid molecules in a host cell. The step of expressing the nucleic acid molecule may be a step in which a host cell anchoring the nucleic acid molecules or a recombinant vector carrying same is cultured. The method may further include a step of separating and/or purifying an antibody from the medium after the expressing step.

**Technical Solution**

Provided herein an anti-GITR antibody binding GITR or an antigen-binding fragment, and medicinal uses thereof. The anti-GITR antibody or the antigen-binding fragment thereof functions as an agonist for GITR to activate immunity (e.g., enhancement of effector T cell function, regulation of Treg activity, increased cytokine secretion, etc.), thus finding applications as various immune activators and/or immunotherapeutics.

As used herein, the term "glucocorticoid-induced TNFR-related protein" (GITR) refers to a member of the TNF-receptor superfamily that binds to a GITR ligand (GITRL). GITR is also known as tumor necrosis factor receptor superfamily member 18 (TNFRSF18), AITR, or CD357. The GITR includes not only GITR naturally expressed by cells, but also any variant or isoform thereof. In an embodiment, the GITR may be human GITR, or an isotype as expressed by UniProt Accession No. Q9Y5U5-1 (NCBI Accession No. NP_004186.1; encoded by NM_004195.3), NCBI Accession No. NP_683699.1 (encoded by NM_148901.2), and NCBI Accession No. NP_683700.1 (encoded by NM_148902.1), but with no limitations thereto.

Below, a detailed description will be given of the present disclosure.

Antibody or Antigen-Binding Fragment

An aspect provides an anti-GITR antibody binding to GITR or an antigen-binding fragment thereof.

The anti-GITR antibody or antigen-binding fragment thereof may include:

a polypeptide including the amino acid sequence of SEQ ID NO: 1 (CDR-H1),
a polypeptide including the amino acid sequence of SEQ ID NO: 2 (CDR-H2),
a polypeptide including the amino acid sequence of SEQ ID NO: 3 (CDR-H3),
a polypeptide including the amino acid sequence of SEQ ID NO: 4 (CDR-L1),
a polypeptide including the amino acid sequence of SEQ ID NO: 5 (CDR-L2),
and
a polypeptide including the amino acid sequence of SEQ ID NO: 6 (CDR-L3).

In an embodiment, the anti-GITR antibody or antigen-binding fragment thereof may include:

a polypeptide including the amino acid sequence of SEQ ID NO: 7, 8, or 9 (CDR-H1),
a polypeptide including the amino acid sequence of SEQ ID NO: 2 (CDR-H2),
a polypeptide including the amino acid sequence of SEQ ID NO: 3 (CDR-H3),
a polypeptide including the amino acid sequence of SEQ ID NO: 4 (CDR-L1),
a polypeptide including the amino acid sequence of SEQ ID NO: 10, 11, or 12 (CDR-L2), and
a polypeptide including the amino acid sequence of SEQ ID NO: 6 (CDR-L3).

In an embodiment, the anti-GITR antibody or antigen-binding fragment thereof may include:

(1) CDR-H1 including the amino acid sequence of SEQ ID NO: 7, CDR-H2 including the amino acid sequence of SEQ ID NO: 2, CDR-H3 including the amino acid sequence of SEQ ID NO: 3, CDR-L1 including the amino acid sequence of SEQ ID NO: 4, CDR-L2 including the amino acid sequence of SEQ ID NO: 10, and CDR-L3 including the amino acid sequence of SEQ ID NO: 6;
(2) CDR-H1 including the amino acid sequence of SEQ ID NO: 8, CDR-H2 including the amino acid sequence of SEQ ID NO: 2, CDR-H3 including the amino acid sequence of SEQ ID NO: 3, CDR-L1 including the amino acid sequence of SEQ ID NO: 4, CDR-L2 including the amino acid sequence of SEQ ID NO: 11, and CDR-L3 including the amino acid sequence of SEQ ID NO: 6; or
(3) CDR-H1 including the amino acid sequence of SEQ ID NO: 9, CDR-H2 including the amino acid sequence of SEQ ID NO: 2, CDR-H3 including the amino acid sequence of SEQ ID NO: 3, CDR-L1 including the amino acid sequence of SEQ ID NO: 4, CDR-L2 including the amino acid sequence of SEQ ID NO: 12, and CDR-L3 including the amino acid sequence of SEQ ID NO: 6.

Herein, the complementarity determining region (CDR) is generally determined as defined according to the kabat system.
In an embodiment, the six CDRs (CDR-L1, CDR-L2, CDR-L3, CDR-H1, CDR-H2, and CDR-H3) that may be included in the anti-GITR antibody or antigen-binding fragment thereof provided herein are summarized in Table 1, below.

TABLE 1

|  | Sequence | SEQ ID NO: |
| --- | --- | --- |
| CDR-H1 | GYWIX(X = E, N, or D) | 1 |
| CDR-H1 | GYWIE | 7 |
| CDR-H1 | GYWIN | 8 |
| CDR-H1 | GYWID | 9 |
| CDR-H2 | EILPGSGATYYSENFKG | 2 |
| CDR-H3 | KGGYYAMDY | 3 |
| CDR-L1 | TASSSVSSSYFH | 4 |
| CDR-L2 | STSNLXS (X = A, Q, or E) | 5 |
| CDR-L2 | STSNLAS | 10 |
| CDR-L2 | STSNLQS | 11 |
| CDR-L2 | STSNLES | 12 |
| CDR-L3 | HLYHRSPRT | 6 |
| (In Table 1, CDR-H1, CDR-H2, and CDR-H3 each stand for a heavy chain complementarity determining region and CDR-L1, CDR-L2, and CDR-L3 each stand for a light chain complementarity determining region) | | |

In an embodiment, the anti-GITR antibody or antigen-binding fragment thereof may include:

a heavy chain variable region including CDR-H1 of SEQ ID NO: 1 (e.g., SEQ ID NO: 7, 8, or 9), CDR-H2 of SEQ ID NO: 2, and CDR-H3 of SEQ ID NO:3, and
a light chain variable region including CDR-L1 of SEQ ID NO:4, CDR-L2 of SEQ ID NO: 5 (e.g., SEQ ID NO: 10, 11, or 12), and CDR-L3 of SEQ ID NO: 6.

In an embodiment, the anti-GITR antibody or antigen-binding fragment thereof may include:

(1) a heavy chain variable region composed of CDR-H1 including the amino acid sequence of SEQ ID NO: 7, CDR-H2 including the amino acid sequence of SEQ ID NO: 2, and CDR-H3 including the amino acid sequence of SEQ ID NO: 3, and
a light chain variable region composed of CDR-L1 including the amino acid sequence of SEQ ID NO: 4, CDR-L2 including the amino acid sequence of SEQ ID NO: 10, and CDR-L3 including the amino acid sequence of SEQ ID NO: 6;
(2) a heavy chain variable region composed of CDR-H1 including the amino acid sequence of SEQ ID NO: 8, CDR-H2 including the amino acid sequence of SEQ ID NO: 2, and CDR-H3 including the amino acid sequence of SEQ ID NO: 3, and
a light chain variable region composed of CDR-L1 including the amino acid sequence of SEQ ID NO: 4, CDR-L2 including the amino acid sequence of SEQ ID NO: 11, and CDR-L3 including the amino acid sequence of SEQ ID NO: 6; or
(3) a heavy chain variable region composed of CDR-H1 including the amino acid sequence of SEQ ID NO: 9, CDR-H2 including the amino acid sequence of SEQ ID NO: 2, and CDR-H3 including the amino acid sequence of SEQ ID NO: 3, and
a light chain variable region composed of CDR-L1 including the amino acid sequence of SEQ ID NO: 4, CDR-L2 including the amino acid sequence of SEQ ID NO: 12, and CDR-L3 including the amino acid sequence of SEQ ID NO: 6.

In an embodiment, the anti-GITR antibody or the antigen-binding fragment thereof may include:

a heavy chain variable region including the amino acid sequence of SEQ ID NO: 13, 14, 15, 16, 17, or 18; and
a light chain variable region including the amino acid sequence of SEQ ID NO: 19, 20, 21, 22, 23, or 24.

In an embodiment, the anti-GITR antibody or the antigen-binding fragment thereof may include:

the heavy chain variable region of SEQ ID NO: 13 and the light chain variable region of SEQ ID NO: 19;
the heavy chain variable region of SEQ ID NO: 14 and the light chain variable region of SEQ ID NO: 20;
the heavy chain variable region of SEQ ID NO: 15 and the light chain variable region of SEQ ID NO: 21;
the heavy chain variable region of SEQ ID NO: 16 and the light chain variable region of SEQ ID NO: 22;
the heavy chain variable region of SEQ ID NO: 17 and the light chain variable region of SEQ ID NO: 23; or
the heavy chain variable region of SEQ ID NO: 18 and the light chain variable region of SEQ ID NO: 24.

Depending on the situation (for example, when produced recombinantly), the heavy chain variable region and/or light chain variable region may further include an appropriate signal sequence at the N-terminus.

Amino acid sequences of the heavy chain variable region and light chain variable region that may be included in the anti-GITR antibody or antigen-binding fragment thereof provided herein are given in Table 2, below:

TABLE 2

| | Amino acid sequence (N→C) | SEQ ID NO: |
|---|---|---|
| Heavy chain variable region | QVQLQQSGAELMKPGASVKLSCKTTGYRFT**GYWIE**WVKQRPGHGLEWIG**EILPGSGATYYSENFKG**KATFTADTSSNTAYMQVSSLTTGDSAIYYCAR**KGGYYAMDY**WGQGTSVTVSS | 13 |
| Heavy chain variable region | QVQLVQSGAELKKPGASVKLSCKTTGYRFT**GYWIE**WVRQPPGKGLEWIG**EILPGSGATYYSENFKG**KATITADTSTNTAYMEVSSLRSGDSAIYYCAR**KGGYYAMDY**WGQGTSVTVSS | 14 |

(continued)

| | Amino acid sequence (N→C) | SEQ ID NO: |
|---|---|---|
| Heavy chain variable region | QVQLVQSGAEVKKPGASVKLSCKTTGYRFT**GYWIE**WVRQPPGKGLEWIG**EILPGSGATYYSENFKG**KATITADTSTNTAYMEVSSLRSEDTAIYYCAR**KGGYYAMDY**WGQGTLVTVSS | 15 |
| Heavy chain | QVQLVQSGAEVKKPGASVKVSCKTTGYRFT**GYWIE**WVRQP | 16 |
| variable region | PGKGLEWIG**EILPGSGATYYSENFKG**KVTITADTSTDTAYMELSSLRSEDTAIYYCAR**KGGYYAMDY**WGQGTLVTVSS | |
| Heavy chain variable region | QVQLVQSGAEVKKPGASVKVSCKTTGYRFT**GYWIN**WVRQPPGKGLEWIG**EILPGSGATYYSENFKG**KVTITADTSTDTAYMELSSLRSEDTAIYYCAR**KGGYYAMDY**WGQGTLVTVSS | 17 |
| Heavy chain variable region | QVQLVQSGAEVKKPGASVKVSCKTTGYRFT**GYWID**WVRQPPGKGLEWIG**EILPGSGATYYSENFKG**KVTITADTSTDTAYMELSSLRSEDTAIYYCAR**KGGYYAMDY**WGQGTLVTVSS | 18 |
| Light chain variable region | QIVLTQSPAIMSASLGERVTMTC**TASSSVSSSYFH**WYQQKPGSSPKLWIY**STSNLAS**GVPARFSGSGSGTSYSLTVSSLEAEDAATYYC**HLYHRSPRT**FGGGTTLDIK | 19 |
| Light chain variable region | QIVLTQSPAIMSASLGERVTMSC**TASSSVSSSYFH**WYQQKPGSAPKLWIY**STSNLAS**GVPDRFSGSGSGTDYTLTVSSLQAEDVATYYC**HLYHRSPRT**FGGGTTVDIK | 20 |
| Light chain variable region | QIVLTQSPATLSLSPGERATMSC**TASSSVSSSYFH**WYQQKPGSAPKLWIY**STSNLAS**GVPDRFSGSGSGTDYTLTISSLQAEDVATYYC**HLYHRSPRT**FGGGTKVEIK | 21 |
| Light chain variable region | EIVLTQSPATLSLSPGERATLSC**TASSSVSSSYFH**WYQQKPGSAPKLWIY**STSNLAS**GVPDRFSGSGSGTDYTLTISSLQAEDVATYYC**HLYHRSPRT**FGGGTKVEIK | 22 |
| Light chain variable region | EIVLTQSPATLSLSPGERATLSC**TASSSVSSSYFH**WYQQKPGKAPKLWIY**STSNLQS**GVPDRFSGSGSGTDYTLTISSLQAEDVATYYC**HLYHRSPRT**FGGGTKVEIK | 23 |
| Light chain variable region | EIVLTQSPATLSLSPGERATLSC**TASSSVSSSYFH**WYQQKPGKAPKLWIY**STSNLES**GVPDRFSGSGSGTDYTLTISSLQAEDVATYYC**HLYHRSPRT**FGGGTKVEIK | 24 |
| (In Table 2, the underlined regions represent CDR1, CDR2, and CDR3 in heavy and light chains, sequentially) | | |

In an embodiment, the anti-GITR antibody or antigen-binding fragment thereof provided herein may bind to one or more amino acids within the GITR protein, for instance, within the human GITR protein (amino acid residues 26-241 in SEQ ID NO: 33; where amino acid residues 1-25 are the signal sequence), and specifically to one or more of the amino acid residues 92 to 132 on the sequence. For example, the anti-GITR antibody or antigen-binding fragment thereof may bind to at least one selected from the regions of amino acid residues 92-96 (SGTFS, SEQ ID NO: 34), 107-116 (TDCTQFGFLT, SEQ ID NO: 35), and 122-132 (KTHNAVCVPGS, SEQ ID NO: 36) or at least one amino acid residue within the regions, e.g., at least one amino acid residue selected from the group consisting of 92S (92nd amino acid residue, Ser(S), and the same interpretation applies hereafter), 96S, 107T, 110T, 116T, 122K, and 132S, but with no limitations thereto (the amino acid residue positions of the human GITR are counted starting from the 26th amino acid residue, with the signal sequence (1-25) excluded.)

[Human GITR (UniProt Q9Y5U5-1, SEQ ID NO: 33, the region of amino acid residues 1-25 (in italics) is a signal sequence)]

*MAQHGAMGAFRALCGLALLCALSLG*QRPTGGPGCGPGRLLLGTGTDARC

CRVHTTRCCRDYPGEECCSEWDCMCVQPEFHCGDPCCTTCRHHPCPPGQGVQ

SQGKFSFGFQCIDCA<u>SGTFS</u>GGHEGHCKPW<u>TDCTQFGFLT</u>VFPGN<u>KTHNAVCVP</u>

<u>GS</u>PPAEPLGWLTVVLLAVAACVLLLTSAQLGLHIWQLRSQCMWPRETQLLLEVPP

STEDARSCQFPEEERGERSAEEKGRLGDLWV

As described herein, the term "antibody" may refer to a protein that specifically binds to a specific antigen, and may be a protein produced by stimulation of an antigen in the immune system, or a protein produced by chemical synthesis or recombinant production, with no specific limitation. The antibody may be non-naturally occurring, for example, produced by recombinant or synthetic production. The antibody may be an animal antibody (e.g., a mouse antibody, etc.), a chimeric antibody, a humanized antibody, or a human antibody. The antibody may be a monoclonal or polyclonal antibody.

In the anti-GITR antibody or antigen-binding fragment thereof provided herein, the portion, except for the heavy-chain CDR and light-chain CDR portions or the heavy-chain variable and light-chain variable regions as defined above, may be derived from any subtype of immunoglobulin (e.g., IgA, IgD, IgE, IgG (IgG1, IgG2, IgG3, IgG4), IgM, and the like), and, for example, derived from the framework portions, and/or light-chain constant region and/or heavy-chain constant region. In an embodiment, the anti-GITR antibody provided herein may be an antibody in a form of human IgG, for example, IgG1, IgG2, IgG3, or IgG4, but not be limited thereto.

An intact antibody (e.g., IgG type) has a structure with two full-length light chains and two full-length heavy chains, in which each light chain is linked to a corresponding heavy chain via a disulfide bond. The constant region of an antibody is divided into a heavy-chain constant region and a light-chain constant region. The heavy-chain constant region is of a gamma ($\gamma$), mu ($\mu$), alpha ($\alpha$), delta ($\delta$), or epsilon ($\varepsilon$) type, and has gamma1 ($\gamma$1), gamma2 ($\gamma$2), gamma3 ($\gamma$3), gamma4 ($\gamma$4), alpha1 ($\alpha$1) or alpha2 ($\alpha$2) as its subclass. The light chain constant region is of either a kappa ($\kappa$) or lambda ($\lambda$) type.

In an embodiment, the anti-GITR antibody provided herein may include a constant region of IgG as the heavy chain constant region and a kappa constant region as the light chain constant region, but with no limitations thereto.

In an embodiment, the constant region of IgG (e.g., human IgG1) may be a wild type. In another embodiment, the constant region of IgG may be a variant that has the mutation N298A (the amino acid N at position 298 substituted by A) on the human IgG1, but with no limitations thereto.

As used herein, the term "heavy chain" may be intended to encompass a full-length heavy chains and fragments thereof, wherein the full-length heavy chain includes a variable region $V_H$ including amino acid sequences sufficient to provide specificity to antigens, three constant regions $C_{H1}$, $C_{H2}$, and $C_{H3}$, and a hinge. The term "light chain" is intended to encompass full-length light chains and fragments thereof, wherein the full-length light chain includes a variable region $V_L$ including amino acid sequences sufficient to provide specificity to antigens, and a constant region $C_L$.

The term "complementarity determining region" (CDR) refers to a portion that confers antigen-binding specificity in a variable region of an antibody and refer to an amino acid sequence found in a hypervariable region of a heavy chain or a light chain of immunoglobulin. The heavy and light chains may each include three CDRs (CDRH1, CDRH2, and CDRH3; and CDRL1, CDRL2, and CDRL3). The CDR may provide contacting residues that play an important role in the binding of an antibody to its antigen or an epitope of the antigen. As used herein, the terms "specifically binding" and "specifically recognizing" may have the same general meaning as known to one of ordinary skill in the art, and indicate that an antibody and an antigen specifically interact with each other to lead to an immunological reaction.

The complementarity determining region (CDR) described herein is determined as defined according to the kabat system.

In this disclosure, unless particularly stated, the term "antibody" may be understood to include an antigen-binding fragment of an antibody having antigen-binding ability.

The term "antigen-binding fragment" used herein may refer to a polypeptide in any type, which includes a portion (e.g., 6 CDRs as defined herein) capable of binding to an antigen, and, for example, may be scFv, scFv-Fc, (scFv)$_2$, Fab, Fab', or F(ab')$_2$, but is not limited thereto.

Among the antigen-binding fragments, Fab has a structure composed of variable regions of light and heavy chains, the constant region of a light chain, and the first constant region (C$_{H1}$) of a heavy chain, with one antigen-binding site retained.

Fab' is different from Fab in that Fab' includes a hinge region having at least one cysteine residue at the C-terminal of the heavy chain C$_{H1}$ domain.

F(ab')$_2$ antibody is formed through disulfide bridging of the cysteine residues in the hinge region of Fab'. Fv is a minimal antibody fragment composed of only a heavy chain variable region and a light chain variable region. Recombination techniques of generating an Fv fragment are widely known in the art.

Two-chain Fv includes a heavy chain variable region and a light chain variable region which are linked to each other by a non-covalent bond. Single-chain Fv generally includes a heavy-chain variable region and a light-chain variable region which are linked to each other by a covalent bond via a peptide linker or directly linked at the C-terminals to have a dimer structure like two-chain Fv.

The antigen-binding fragments may be obtained using protease (for example, Fab may be obtained by restrictively cleaving a whole antibody with papain, and an F(ab')$_2$ fragment may be obtained by cleaving with pepsin), or may be prepared by using a genetic recombination technique.

The term "hinge region" refers to a region between C$_{H1}$ and C$_{H2}$ domains within heavy chain of an antibody, which functions to provide flexibility for the antigen-binding site in the antibody.

The antibody provided herein may be a monoclonal antibody. A monoclonal antibody can be prepared using a method widely known in the art, for example, using a phage display technique. Alternatively, the antibody may be constructed in the form of an animal (e.g., mouse)-derived monoclonal antibody by a conventional method.

Meanwhile, individual monoclonal antibodies can be screened using a typical ELISA (Enzyme-Linked ImmunoSorbent Assay) format, based on the binding potential against the receptor binding domain of GITR. Inhibitory activities can be verified through functional analysis such as competitive ELISA for verifying the molecular interaction of binding assemblies or functional analysis such as a cell-based assay. Then, with regard to monoclonal antibody members selected on the basis of their strong inhibitory activities, their affinities (Kd values) to the receptor binding domain of GITR may be each verified.

The finally selected antibodies can be prepared and used as humanized antibodies as well as human immunoglobulin antibodies in which the remaining parts except for the antigen-binding portion are humanized. Methods for producing humanized antibodies are well known in the art.

An antigen-binding fragment of the anti-GITR antibody provided herein may refer to a fragment which is derived from an anti-GITR antibody and retain antigen (GITR) binding affinity of the anti-GITR antibody. In an embodiment, the antigen-binding fragment may be a polypeptide including the 6 CDRs of an anti-GITR antibody as described above, and, for example, may be scFv, scFv-Fc, scFv-Ck (kappa constant region), scFv-Cλ (lambda constant region), (scFv)$_2$, Fab, Fab', or a F(ab')$_2$, but not be limited thereto. In an embodiment, the antigen-binding fragment may be a fusion polypeptide in which scFv or scFv is fused to the Fc region of an immunoglobulin (e.g., IgG1, IgG2, IgG3, IgG4, etc.) (scFv-Fc) or to a light chain constant region (e.g., kappa or lambda) (scFv-Ck or scFv-Cλ), but with no limitations thereto.

In this disclosure, an antibody (for example, CDR, variable region, or heavy chain/light chain, antigen-binding fragment etc.) "including (comprising) a specific amino acid sequence" or "consisting of or expressed a specific amino acid sequence" refers to all cases which the amino acid sequence is essentially included, and/or an insignificant mutation (for example, substitution, deletion, and/or addition of amino acid residue(s)) that does not affect antibody activity (for example, antigen binding affinity, pharmacological activity) is introduced into the amino acid sequence.

The anti-GITR antibody or antigen-binding fragment thereof provided herein may have a binding affinity (K$_D$) to GITR (for example, human GITR) of 10 mM or less, 5 mM or less, 1 mM or less, 0.5 mM or less, 0.2 mM or less, 0.1 mM or less, 0.05 mM or less, 0.01 mM or less, 0.005 mM or less, or 0.001 mM or less, for example, 0.0001 nM to 10 mM, 0.0005 nM to 10 mM, 0.001 nM to 10 mM, 0.005 nM to 10 mM, 0.01 nM to 10 mM, 0.05 nM to 10 mM, 0.1 nM to 10 mM, 0.5 nM to 10 mM, 1 nM to 10 mM, 0.0001 nM to 5 mM, 0.0005 nM to 5 mM, 0.001 nM to 5 mM, 0.005 nM to 5 mM, 0.01 nM to 5 mM, 0.05 nM to 5 mM, 0.1 nM to 5 mM, 0.5 nM to 5 mM, 1 nM to 5 mM, 0.0001 nM to 1 mM, 0.0005 nM to 1 mM, 0.001 nM to 1 mM, 0.005 nM to 1 mM, 0.01 nM to 1 mM, 0.05 nM to 1 mM, 0.1 nM to 1 mM, 0.5 nM to 1 mM, 1 nM to 1 mM, 0.0001 nM to 0.5mM, 0.0005 nM to 0.5mM, 0.001 nM to 0.5mM, 0.005 nM to 0.5mM, 0.01 nM to 0.5 mM, 0.05 nM to 0.5 mM, 0.1 nM to 0.5 mM, 0.5 nM to 0.5 mM, 1 nM to 0.5 mM, 0.0001 nM to 0.2 mM, 0.0005 nM to 0.2 mM, 0.001 nM to 0.2 mM, 0.005 nM to 0.2 mM, 0.01 nM to 0.2 mM, 0.05 nM to 0.2 mM, 0.1 nM to 0.2 mM, 0.5 nM to 0.2 mM, 1 nM to 0.2 mM, 0.0001 nM to 0.1 mM, 0.0005 nM to 0.1 mM, 0.001 nM to 0.1 mM, 0.005 nM to 0.1 mM, 0.01 nM to 0.1 mM, 0.05 nM to 0.1 mM, 0.1 nM to 0.1 mM, 0.5 nM to 0.1 mM, 1 nM to 0.1 mM, 0.0001 nM to 0.05 mM, 0.0005 nM to 0.05 mM, 0.001 nM to 0.05 mM, 0.005 nM to 0.05 mM, 0.01 nM to 0.05 mM, 0.05 nM to 0.05 mM, 0.1 nM to 0.05 mM, 0.5 nM to 0.05 mM, 1 nM to 0.05 mM, 0.0001 nM to 0.01 mM, 0.0005 nM to 0.01 mM, 0.001 nM to 0.01 mM, 0.005 nM to 0.01 mM, 0.01 nM to 0.01 mM, 0.05 nM to 0.01 mM, 0.1 nM to 0.01 mM, 0.5 nM to 0.01 mM, or 1 nM to 0.01mM, as measured by surface plasmon resonance (SPR; e.g., Biacore), but with no limitations thereto.

Another aspect provides a polypeptide molecule including a heavy chain complementarity determining region (CDR-H1, CDR-H2, CDR-H3, or a combination thereof), a light chain complementarity determining region (CDR-L1, CDR-L2, CDR-L3, or a combination thereof), a combination thereof; or heavy chain variable region, light chain variable region, or a combination thereof in the anti- GITR antibody described in the foregoing.

The polypeptide molecule may be used in preparing an antibody as a precursor of antibody, or comprised in a protein scaffold having an antibody-like structure (e.g., peptibody, nanobody), a bispecific antibody, or a multispecific antibody, as a component thereof (e.g., CDR or variable region).

As used herein, the term "peptibody" (peptide + antibody) refers to a fusion protein having similar framework and functions to an antibody, wherein a peptide is fused with the whole or a part of a constant region of an antibody, such as Fc region, and serves as an antigen binding site (heavy chain and/or light chain CDR or variable regions).

The term "nanobody", as used herein, is called a single-domain antibody, refers to an antibody fragment including a single variable domain of an antibody in a monomer form, which exhibits characteristics of selectively binding to a specific antigen in a similar manner to that for an antibody having an intact structure. The molecular weight of the nanobody is generally about 12 kDa to about 15 kDa, which is very little when compared to the normal molecular weight (about 150 kDa or about 160 kDa) of an intact antibody (including two heavy chains and two light chains) and in some cases it is smaller than an Fab fragment or scFv fragment.

The term "multi-specific antibody" (including bispecific antibody), as used herein, refers to an antibody recognizing and/or binding to two or more different antigens, or recognizing and/or binding to different sites of the same antigen, and one antigen binding site of the multi-specific antibody may include the polypeptide, antibody, or antigen-binding fragment described above to bind to GITR.

The polypeptide, antibody, or antigen-binding fragment provided herein, which binds to GITR, may be used in the form of a conjugate with at least one selected from among a useful polymer, a label, and the like.

The useful polymer may be, for example, a non-protein polymer that increases the in vivo half-life of a polypeptide, antibody, and/or antigen-binding fragment, and may be at least one hydrophilic polymer selected from the group among polyethylene glycol (PEG) (e.g., 2 kDa, 5 kDa, 10 kDa, 12 kDa, 20 kDa, 30 kDa or 40 kDa PEG), dextran, monomethoxypolyethylene glycol (mPEG), and the like, but with no limitations thereto.

The label may be at least one radionucleotide or fluorescent or chemilluminescent small chemical selected from among rare earth chelates, fluorescein and its derivatives, rhodamine and its derivatives, isothiocyanate, phycoerythrin, phycocyanin, allophycocyanin, o-phthaladehyde, fluorescamine, 152Eu, dansyl, umbelliferone, luciferin, a luminal label, an isoluminal label, an aromatic acridinium ester label, an imidazole label, an acridimium salt label, an oxalate ester label, an aequorin label, 2,3-dihydrophthalazinediones, biotin/avidin, spin labels, and stable free radicals, but with no limitations thereto.

Medicinal Use

The anti-GITR antibody or antigen-binding fragment thereof provided herein acts as an agonist for GITR (GITR activation) and functions to activate immunity (e.g., enhancement of effector T cell function, regulation of Treg activity, increased cytokine secretion, etc.). Thus, the anti-GITR antibody or antigen-binding fragment thereof can be applied to immunopotentiation and can find advantageous applications in the prevention and/or treatment of immune-related diseases, particularly cancer.

Another aspect provides an immunopotentiator or an immunopotent pharmaceutical composition, each including the anti-GITR antibody or antigen-binding fragment thereof as an active ingredient.

Another aspect provides a pharmaceutical composition including the anti-GITR antibody or antigen-binding fragment thereof as an active ingredient for prevention and/treatment of an immune-related disease.

Another aspect provides an anticancer agent or a pharmaceutical composition for the prevention and/or treatment of cancer, each including the anti-GITR antibody or antigen-binding fragment thereof as an active ingredient.

Another aspect provides an immunopotentiation method including a step of administering a pharmaceutically effective amount of the anti-GITR antibody or antigen-binding fragment thereof to a subject in need of immunopotentiation. The immunopotentiation method may further include a step of identifying a subject in need of immunopotentiation before the administering step.

Another aspect provides a method for preventing and/or treating an immune-related disease, the method including a step of administering a pharmaceutically effective amount of the anti-GITR antibody or antigen-binding fragment thereof to a subject in need of immune-related disease prevention and/or treatment. The method for preventing and/or treating an immune-related disease may further include a step of identifying a subject in need of immune-related disease prevention and/or treatment before the administering step.

Another aspect provides a method for preventing and/or treating cancer, the method including a step of administering a pharmaceutically effective amount of the anti-GITR antibody or antigen-binding fragment thereof to a subject in need of cancer prevention and/or treatment. The method for preventing and/or treating cancer may further include a step of identifying a subject in need of cancer prevention and/or treatment before the administering step.

Another aspect provides a use of the anti-GITR antibody or antigen-binding fragment thereof for immunopotentiation or for preparing an immunopotentiator.

Another aspect provides a use of the anti-GITR antibody or antigen-binding fragment thereof for preventing and/or treating an immune-related disease or for preparing a drug for the prevention and/or treatment of an immune-related disease.

Another aspect provides a use of the anti-GITR antibody or antigen-binding fragment thereof for preventing and/or treating cancer or for preparing a drug for the prevention and/or treatment of cancer.

As used herein, the term "immunopotentiation" (or immunoenhancement) means inducing an initial immune response to an antigen or increasing an existing immune response and can be interchangeably used with the terms immunostimulation, immunoaugmentation, immunoactivation, and the like. In an embodiment, immunopotentiation may be performed by at least one selected from among functional enhancement (activation) and/or proliferation of immune cells (effector T cells such as cytotoxic T cells; $CD3_+$ T cells, $CD4_+$ T cells, $CD8_+$ T cells, etc.), inactivation and/or depletion of regulatory T (Treg) cells, increased production and/or secretion of immune proteins (e.g., cytokines, etc.), but with no limitations thereto.

As used herein, the term "immune-related disease" encompasses all diseases caused by impairment and/or insufficient activity of the immune system. Examples of the immune-related disease includes cancer, infectious diseases, autoimmune diseases, inflammatory diseases, and the like, but are not limited thereto.

The cancer may be solid cancer or hematological cancer, but is not limited thereto, and may be at least one selected from the group consisting of squamous cell carcinoma, lung cancer (e.g., small cell lung cancer, non-small cell lung cancer, adenocarcinoma of the lung, squamous cell carcinoma of the lung, etc.), peritoneal cancer, skin cancer, melanoma ( e.g., skin or intraocular melanoma, etc.), rectal cancer, esophageal cancer, small intestine cancer, endocrine cancer, thyroid cancer, parathyroid cancer, adrenal cancer, soft tissue sarcoma, urethral cancer, chronic or acute leukemia, lymphocytic lymphoma, liver cancer, gastric cancer, pancreatic cancer, glioblastoma, cervical cancer, ovarian cancer, bladder cancer, breast cancer, colon cancer (e.g., colon cancer, rectal cancer, colorectal cancer, etc.), endometrial or uterine cancer, salivary gland cancer, kidney cancer, prostate cancer, vulvar cancer, head and neck cancer, brain cancer, and osteosarcoma.

The prophylactic and/or therapeutic effects on cancer include all effects of inhibiting the generation and/or growth of cancer cells, and suppressing the aggravation of cancer due to migration, invasion, metastasis, etc.

The infectious diseases, autoimmune diseases, and inflammatory diseases may be selected from all infectious diseases, autoimmune diseases, and inflammatory diseases that can be treated, alleviated, and/or prevented by the immunopotentiation described in the foregoing (e.g., functional potentiation (activation) and/or proliferation of immune cells (effector T cells, such as cytotoxic T cells; $CD3_+$ T cells, $CD4_+$ T cells, $CD8_+$ T cells, etc.), activity inhibition and/or depletion of regulatory T (Treg) cells, increased production and/or secretion of immune proteins (e.g., cytokines (IL-2, IFN-gamma and the like), etc.). For instance, infectious disease is a generic term for diseases that arise when pathogens, such as viruses, bacteria, fungi, and parasites, spread and invade into a living organism (e.g., animals including humans). They may be infections or diseases caused by one or more pathogens selected from group consisting of viruses, bacteria, fungi, parasites, etc. The autoimmune disease may be selected from a group consisting of rheumatoid arthritis, type 1 diabetes, Crohn's disease, ulcerative colitis, Behcet's syndrome, lupus, Sjogren's syndrome, myasthenia gravis, scleroderma, hypothyroidism, hyperthyroidism, psoriasis, vitiligo, multiple sclerosis, autoimmune hepatitis, autoimmune nephritis, autoimmune pancreatitis, autoimmune encephalitis, cytokine storm, etc., but are not limited thereto. The term "inflammatory disease" refers to inflammation (e.g., chronic inflammation or acute inflammation) or a disease caused by inflammation. Examples of the inflammatory disease include heart inflammation (e.g., coronary artery disease, angina, myocardial infarction, pericarditis, myocarditis, etc.), vascular inflammation (e.g., atherosclerosis, vasculitis, disseminated intravascular coagulation (DIC), immune thrombocytopenic purpura (ITP), thrombotic thrombocytopenic purpura (TTP), anemia, etc.), upper respiratory tract inflammation (e.g. acute nasopharyngitis, allergic rhinitis, sinusitis, pharyngitis, tonsillitis, laryngitis, etc.), lower respiratory tract and/or lung inflammation (e.g. bronchitis, bronchiectasis, asthma, chronic pulmonary active pulmonary disease (COPD), pneumonia, interstitial lung disease, tuberculosis, etc.), upper gastrointestinal tract inflammation (e.g. gastritis, esophagitis, etc.), lower gastrointestinal tract (e.g., enteritis, ulcerative colitis, Crohn's disease, celiac disease, diverticulitis, irritable bowel syndrome, appendicitis, perianal fistula, etc.), inflammation of the liver, biliary tract and/or pancreas (e.g., hepatitis, fatty liver, cholangitis, cholecystitis, pancreatitis, type 1 diabetes, etc.), kidney (upper urinary tract) inflammation (e.g., pyelonephritis, glomerulonephritis, urinary tract infections, etc.), lower urinary tract inflammation (e.g., urinary tract infections, ureteritis, urethritis, cystitis, prostatitis/chronic pelvic pain syndrome, etc.), thyroid and/or parathyroid inflammation (e.g., thyroiditis, parathyroiditis, etc.), adrenal inflammation (e.g., adrenalitis, etc.), genital inflammation (e.g., pelvic inflammatory diseases, oophoritis, orchitis, epididymitis, etc.), bone and/or joint inflammation (e.g., osteoarthritis, rheumatoid arthritis, osteomyelitis, synovitis, etc.), skin inflammation (e.g., skin: cellulitis, erysipelas, tinea versicolor, athlete's foot, acne etc.), muscle inflammation (e.g., myositis, etc.), brain inflammation (e.g., encephalitis, major depressive disorder, etc.), nerve inflammation (e.g., neuritis in various parts such as eyes, ears, etc., complex regional pain syndrome, Guillain-Barre syndrome, etc.), eye inflammation (e.g., stye, uveitis, conjunctivitis, etc.), ear inflammation (e.g., otitis media, mastoiditis, etc.), oral inflammation (e.g., stomatitis, periodontitis, gingivitis, etc.), systemic inflammation (e.g., systemic inflammatory response syndrome (sepsis), metabolic syndrome-related diseases, etc.), peritonitis, reperfusion injury, transplant rejection response, and hypersensitivity, but are not limited thereto.

The anti-GITR antibody, antigen-binding fragment thereof, and/or pharmaceutical composition including same provided herein may be administered to any animal or cell, for example, animals selected from mammals including primates such as humans and monkeys, rodents such as rats, mice, and the like., or cells, tissues, body fluids (e.g., sera) derived (isolated) from the animals, or cultures thereof, e.g., cells, tissues, body fluid (sera) isolated from humans.

The pharmaceutical composition may include a pharmaceutically acceptable carrier in addition to the anti-GITR antibody or antigen-binding fragment thereof as an active ingredient. The pharmaceutically acceptable carrier is commonly used in the formulation of protein drugs, and may be at least one selected from the group consisting of lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, but with no limitations thereto. The pharmaceutical composition may further include at least one selected from the group consisting of diluents, excipients, lubricants, wetting agents, sweeteners, flavoring agents, emulsifiers, suspending agents, preservatives, and the like, which are commonly used in the preparation of pharmaceutical compositions. The anti-GITR antibody, antigen-binding fragment thereof and/or pharmaceutical composition can be administered via an oral or parenteral route. For parenteral administration, intravenous injection, subcutaneous injection, intramuscular injection, intraperitoneal injection, intradermal administration, topical administration, intranasal administration, intrapulmonary administration, intrarectal administration, etc. may be taken. Since proteins or peptides are digested when administered orally, the active ingredient in the compositions for oral administration may be coated or formulated to prevent digestion in stomach.

In addition, the anti-GITR antibody, antigen-binding fragment thereof, and/or pharmaceutical composition may be in the form of a solution, suspension, syrup or emulsion in an oil or aqueous medium, or may be formulated into extracts, pulvis, powders, granules, tablets or capsules, injections, etc. The composition may additionally include a dispersing agent or a stabilizer for formulation.

The content of the anti-GITR antibody or antigen-binding fragment thereof or the dosage thereof in the pharmaceutical composition may be determined, depending on various factors, such as the formulation method, administration method, patient's age, weight, sex, pathology, meals, and administration time, administration interval, administration route, excretion rate, response sensitivity, etc. The pharmaceutical composition may be administered at a daily dose, based on the active ingredient (the anti-GITR antibody or antigen-binding fragment), of 0.00001 to 1000 mg/kg, 0.00001 to 500 mg/kg, 0.00001 to 100 mg/kg, 0.00001 to 50 mg/kg, 0.0001 to 1000 mg/kg, 0.0001 to 500 mg/kg, 0.0001 to 100 mg/kg, 0.0001 to 50 mg/kg, 0.001 to 1000 mg/kg, 0.001 to 500 mg/kg, 0.001 to 100 mg/kg, 0.001 to 50 mg/kg, 0.01 to 1000 mg/kg, 0.01 to 500 mg/kg, 0.01 to 100 mg/kg, 0.01 to 50 mg/kg, 0.1 to 1000 mg/kg, 0.1 to 500 mg/kg, 0.1 to 100 mg/kg, or 0.1 to 50 mg/kg, but with no limitations thereto.

The daily dose may be formulated as one formulation in unit dose form, formulated in appropriate portions, or prepared by placing it in a multi-dose container. In addition, in the present specification, a pharmaceutically effective amount refers to an amount of an active ingredient that can exhibit a desired pharmacological activity of an active ingredient, and may be within the above-described dosage range.

Construction of Polynucleotide and Expression Vector and Production of Antibody

Another aspect provides a nucleic acid molecule encoding the heavy chain complementarity determining region, heavy chain variable region, or heavy chain of the anti-GITR antibody. In an embodiment, the nucleic acid may include the nucleic acid sequence of SEQ ID NO: 8 or 12.

Another aspect provides a nucleic acid encoding the light chain complementarity determining region, light chain variable region, or light chain of the anti-GITR antibody. In an embodiment, the nucleic acid may include the nucleic acid sequence of SEQ ID NO: 10 or 14.

Another aspect provides a recombinant vector provides a recombinant vector carrying a nucleic acid molecule coding for the heavy chain complementarity determining region, heavy chain variable region, or heavy chain of the anti-GITR antibody and a nucleic acid molecule coding for the light chain complementarity determining region, light chain variable region, or light chain of the anti-GITR antibody in combination, or separate recombinant vectors carrying a nucleic acid molecule coding for the heavy chain complementarity determining region, heavy chain variable region, or heavy chain of the anti-GITR antibody and a nucleic acid molecule coding for the light chain complementarity determining region, light chain variable region, or light chain of the anti-GITR antibody, respectively. The recombinant vectors may be expression vectors for expressing the nucleic acid molecules.

Another aspect provides a recombinant cell anchoring the recombinant vector thereat.

As used herein, the term "vector" refers to a means for expressing a target gene in a host cell, as exemplified by a plasmid vector, a cosmid vector, and a viral vector such as a bacteriophage vector, a lentivirus vector, an adenovirus vector, a retrovirus vector, and an adeno-associated virus vector. The recombinant vector may be constructed by manipulating a plasmid (for example, pSC101, pGV1106, pACYC177, ColE1, pKT230, pME290, pBR322, pUC8/9, pUC6, pBD9, pHC79, pIJ61, pLAFR1, pHV14, pGEX series, pET series, pUC19, etc.), a phage (for example, λgt4λB, λ-Charon, λΔz1, M13, etc.), or a virus vector (for example, SV40, etc.), which is commonly used in the art.

In the recombinant vector, the nucleic acid molecule may be operatively linked to a promoter. The term "operatively linked" is intended to pertain to a functional linkage between a nucleotide sequence of interest and an expression regulatory sequence (for example, a promoter sequence). When being "operatively linked", the regulatory element can control the transcription and/or translation of a polynucleotide of interest.

The recombinant vector may be constructed typically as a cloning vector or an expression vector. For recombinant expression vectors, a vector generally available in the relevant art for expressing a foreign protein in plant, animal, or microbial cells may be employed. Various methods well known in the art may be used for the construction of recombinant vectors.

For use in hosts, such as prokaryotic or eukaryotic cells, the recombinant vector may be constructed accordingly. For example, when a vector is constructed as an expression vector for use in a prokaryotic host, the vector typically includes a strong promoter for transcription (e.g., a pL$^\lambda$ promoter, a CMV promoter, a *trp* promoter, a *lac* promoter, a *tac* promoter, a T7 promoter, etc.), a ribosomal binding site for initiating translation, and transcriptional/translational termination sequences. On the other hand, an expression vector for use in a eukaryotic host includes an origin of replication operable in a eukaryotic cell, such as an f1 origin of replication, an SV40 origin of replication, a pMB1 origin of replication, an adeno origin of replication, an AAV origin of replication, and a BBV origin of replication, but is not limited thereto. In addition, the expression vector typically includes a promoter derived from genomes of mammalian cells (for example, metallothionein promoter) or from mammalian viruses (for example, adenovirus late promoter, vaccinia virus 7.5K promoter, SV40 promoter, cytomegalovirus promoter, *tk* promoter of HSV, etc.), and a polyadenylation sequence as a transcription termination sequence.

The recombinant cell may be prepared by introducing the recombinant vector into a suitable host cell. As long as it allows the sequential cloning and expression of the recombinant vector in a stable manner, any host cell known in the art may be employed in the present disclosure. Examples of the prokaryotic host cell available for the present disclosure may be selected from E. coli such as *E. coli* JM109, *E. coli* BL21, *E. coli* RR1, *E. coli* LE392, *E. coli* B, *E. coli* X 1776, *E. coli* W3110, Bacillus spp. such as *Bacillus subtilis* and *Bacillus thuringiensis,* and enterobacteriaceae strains such as *Salmonella typhimurium, Serratia marcescens* and various Pseudomonas species. Eukaryotic host cells that may be used for transformation may selected from, but are not limited to, *Saccharomyces cerevisiae,* insect cells, plant cells, and animal cells, such as Sp2/0, CHO (Chinese hamster ovary) K1, CHO DG44, CHO S, CHO DXB11, CHO GS-KO, PER.C6, W138, BHK, COS-7, 293, HepG2, Huh7, 3T3, RIN, MDCK, etc.

The nucleic acid molecule or a recombinant vector carrying same may be delivered (introduced) into a host cell using a method well known in the relevant art. For example, this delivery may be carried out using a CaCl$_2$ or electroporation method when the host cell is prokaryotic. For eukaryotic host cells, the genetic introduction may be achieved using, but not limited to, microinjection, calcium phosphate precipitation, electroporation, liposome-mediated transfection, or particle bombardment.

To select a transformed host cell, advantage may be taken of a phenotype associated with a selection marker according to methods well known in the art. For example, when the selection marker is a gene conferring resistance to a certain antibiotic, the host cells may be grown in the presence of the antibiotic in a medium to select a transformant of interest.

Another aspect provides a method for producing the anti-GITR antibody or antigen-binding fragment thereof, the method including a step of expressing the nucleic acid molecules in a host cell. The step of expressing the nucleic acid molecule in a host cell may be a step in which a cell anchoring the nucleic acid molecules or a recombinant vector carrying same is cultured. The method may further include a step of separating and/or purifying an antibody or antigen-binding fragment from the medium after the culture step.

**Advantageous Effects**

Serving as an agonist for GITR, the anti-GITR antibody or antigen-binding fragment thereof provided herein has the function of activating immunity (e.g., enhancing effector T cell functions, regulating Treg activity, increasing cytokine secretion, etc.) and thus can find advantageous applications as various immune activators and/or immune therapeutics.

**Description of Drawings**

FIGS. 1a and 1b show sequence alignments of heavy chains (1a) and light chains (1b) of the chimeric and humanized antibodies according to an embodiment.
FIG. 2 shows the epitope mapping results of the anti-GITR antibody according to one embodiment.
FIG. 3a is a plot showing the binding affinity of the anti-GITR antibody according to one embodiment to the antigen thereof (human GITR).
FIG. 3b shows plots of the binding affinity of the anti-GITR antibody according to one embodiment to GITR in human primary immune cells.
FIG. 4 shows the GITR agonistic efficacy of the anti-GITR antibody according to an embodiment.
FIGS. 5a to 5d are graphs showing the cytokine production levels in human primary T cells upon treatment with the anti-GITR antibody according to an embodiment, as analyzed for IL-2 levels in CD4+ T cells (5a), IFN-gamma levels in CD4+ T cells (5b), IL-2 levels in CD8+ T cells (5c), and IFN-gamma levels in CD8+ T cells (5d).
FIG. 6 shows graphs illustrating the enhanced effect of the anti-GITR antibody according to one embodiment on cytokine production in human T cells.
FIGS. 7a and 7b show the proliferative effects of the anti-GITR antibody according to one embodiment on NK cells, as analyzed for proliferation levels of isolated NK cells (7a) and proliferation levels of NK cell-containing PBMCs (7b).
FIG. 8 is a graph showing the T cell proliferation effect of the anti-GITR antibody according to one embodiment.
FIG. 9a is a graph showing the expression level of CD107a on the surface of NK cells after treatment with the anti-GITR antibody according to one embodiment.
FIG. 9b is a graph showing the number of Treg cells after treatment with the anti-GITR antibody according to one embodiment.
FIGS. 10a and10b are plots showing killing effects of the anti-GITR antibody according to an embodiment against tumour cells (ovarian cancer cells)
FIG. 11 shows graphs illustrating increased cytokine production effects of the anti-GITR antibody according to an embodiment in T cells isolated from colorectal cancer patients.
FIG. 12 shows graphs illustrating increased cytokine production effects of the anti-GITR antibody according to an embodiment in T cells isolated from lung cancer patients.
FIG. 13 shows graphs illustrating increased cytokine production effects of the anti-GITR antibody according to an embodiment in T cells isolated from liver cancer patients.
FIG. 14 is a plot of tumour sizes in tumour animal models after treatment with the anti-GITR antibody according to an embodiment.

**Mode for Invention**

A better understanding of the present disclosure may be obtained via the following Examples, which are set forth to illustrate, but are not to be construed to limit, the present disclosure. It is obvious to those skilled in the art the following Examples could be modified without departing the gist of the present invention.

**EXAMPLE 1: Construction of Anti-GITR Antibody**

**1.1. Production of anti-GITR monoclonal antibody**

Ten mice (5 Balb/c and 5 C57/bl6) were immunized with an immunogen (GITR protein, >90% purity, mammalian expressed; Genscript).

The immunization schedule was as follows:

Primary Immunization: s.c. injection 50 ug human GITR protein(Q9Y5U5-1) per animal;
1st Boost: T= 14 days i.p. injection 25 ug human GITR protein per animal;

2nd Boost: T= 28 days s.c. injection 25 ug human GITR protein per animal;

Final Boost: T= 56±7 days i.p. injection 25 ug human GITR protein per animal.

Cell Fusion and Screening

B lymphocytes were collected from the spleen of the immunized mice and were fused with SP2/0 myeloma cells to produce hybridomas. This cell fusion was performed by electro fusion. The fusion efficiency was observed to be approximately 1 hybridoma per 3,000 B cells. All the fused cells obtained from each cell fusion were plated onto 96-well plates. Up to 50 plates were used for each fusion.

Subsequently, primary protein screening and FACS confirmation screening were carried out. The primary protein screening was performed using ELISA against the immunization antigen (soluble GITR protein). FACS was conducted using a cell line (Genscript) that stably expresses GITR, and the positive supernatants from the primary screening results were screened.

Afterwards, ligand binding screening was carried out. The previously screened positive clones were screened by ELISA to check whether these clones block or enhance the binding of GITR to GITRL. Specific positive clones were selected for use in the subsequent one-round subcloning.

Subcloning & Expansion

One round subcloning and hybridoma culture supernatant collection: Each of the monoclonal cells was chosen from the parental clones previously selected for assay purposes. Culture supernatants of each expanded clone from the 24-well plate were collected in a volume of 1 ml, and the antibody concentration in the culture supernatant was measured.

Subcloning: Positive primary clones obtained from each selected fusion were subcloned by limiting dilution to ensure that these subclones were derived from a single cell. These clones were passaged up to 3 generations.

Subcloning Screening: These subclones were screened using ELISA or HTP FACS.

The supernatant from the hybridoma tissue culture was screened against the antigen (Genscript) using 2 rounds of indirect ELISA. A competition assay using indirect ELISA was further tested for ligand binding inhibition on the positive clones. Selected clones were co-cultured with GITR-overexpressing CHO cells, and FACS was performed to test for GITR binding.

Complete Subcloning: For each selected monoclonal derived from cell fusion, subcloning was further conducted over two rounds to obtain stable subclones.

Antibody Sequence Sequencing

Total RNA was isolated from the hybridoma cells using TRIzol® Reagent (Ambion, Cat.15596-026) according to the manufacturer's instructions. Subsequently, the PrimeScript™ 1st Strand cDNA Synthesis Kit (Takara, Cat.6110A) was used according to the manufacturer's guidelines to reverse transcribe the total RNA into cDNA through either universal primers or isotype-specific anti-sense primers. Fragments of the antibody heavy and light chains were amplified and each was cloned into a standard cloning vector. Colony PCR was performed to select clones with the correct size of inserts.

The anti-GITR monoclonal antibody clone representative of the selected clones was analyzed for the amino acid sequences of heavy and light chain variable regions of anti-GITR monoclonal antibody (51B clone) and the results are given in Table 3 below.

TABLE 3

| Variable region | Amino acid sequence (N→C) or nucleic acid sequence (5'→3') | SEQ ID NO: |
|---|---|---|
| Heavy chain variable region | QVQLQQSGAELMKPGASVKLSCKTTGYRFT**GYWIE**WVKQRPGHGL EWIG**EILPGSGATYYSENFKG**KATFTADTSSNTAYMQVSSLTTGDSA IYYCAR**KGGYYAMDY**WGQGTSVTVSS | 13 |
| | CAGGTTCAGCTGCAGCAGTCTGGAGCTGAGCTGATGAAGCCTGG GGCCTCAGTGAAGCTTTCCTGCAAGACTACTGGCTACAGATTCAC T**GGCTACTGGATAGAG**TGGGTAAAACAAAGGCCTGGACATGGCC TTGAGTGGATCGGA**GAGATTTTACCTGGAAGTGGTGCTACTTAC TACAGTGAGAACTTCAAGGGC**AAGGCCACATTCACTGCAGATAC ATCCTCCAACACAGCCTACATGCAAGTCAGCAGCCTGACAACTG GGGACTCTGCCATCTATTATTGTGCAAGA**AAGGGGGGGTACTAT GCTATGGACTAC**TGGGGTCAAGGAACCTCAGTCACCGTCTCCTC A | 27 |
| Light chain variable region | QIVLTQSPAIMSASLGERVTMTC**TASSSVSSSYFH**WYQQKPGSSPKL WIY**STSNLAS**GVPARFSGSGSGTSYSLTVSSLEAEDAATYYC**HLYH RSPRT**FGGGTTLDIK | 19 |
| | CAAATTGTTCTCACCCAGTCTCCAGCAATCATGTCTGCATCTCTAG GGGAACGGGTCACCATGACCTGC**ACTGCCAGCTCAAGTGTAAG TTCCAGTTATTTTCAC**TGGTACCAGCAGAAGCCAGGATCCTCACC CAAACTCTGGATTTAT**AGCACTTCCAACCTGGCTTCT**GGAGTCCC AGCTCGCTTCAGTGGCAGTGGGTCTGGGACCTCTTACTCTCTCAC AGTCAGCAGTTTGGAGGCTGAGGATGCTGCCACTTATTACTGC**C ACCTGTATCATCGTTCCCCACGGACG**TTCGGTGGAGGCACCACG CTGGATATCAAA | 28 |

EP 4 339 209 A1

| Variable region | Amino acid sequence (N→C) or nucleic acid sequence (5'→3') | SEQ ID NO: |
|---|---|---|
| Heavy chain variable region (including signal sequence) | *MEWTWVFLFLLSVTAGVHS*QVQLQQSGAELMKPGASVKLSCKTTG YRFT**GYWIE**WVKQRPGHGLEWIG**EILPGSGATYYSENFKG**KATFTA DTSSNTAYMQVSSLTTGDSAIYYCAR**KGGYYAMDY**WGQGTSVTVS S | 29 |
| | *ATGGAATGGACCTGGGTCTTTCTCTTCCTCCTGTCAGTAACTGCA GGTGTCCACTCC*CAGGTTCAGCTGCAGCAGTCTGGAGCTGAGCT GATGAAGCCTGGGGCCTCAGTGAAGCTTTCCTGCAAGACTACTG GCTACAGATTCACT**GGCTACTGGATAGAG**TGGGTAAAACAAAGG CCTGGACATGGCCTTGAGTGGATCGGA**GAGATTTTACCTGGAAG** **TGGTGCTACTTACTACAGTGAGAACTTCAAGGGC**AAGGCCACAT TCACTGCAGATACATCCTCCAACACAGCCTACATGCAAGTCAGCA GCCTGACAACTGGGGACTCTGCCATCTATTATTGTGCAAGA**AAG** **GGGGGGTACTATGCTATGGACTAC**TGGGGTCAAGGAACCTCAGT CACCGTCTCCTCA | 30 |

| Variable region | Amino acid sequence (N→C) or nucleic acid sequence (5'→3') | SEQ ID NO: |
|---|---|---|
| Light chain variable region (including signal sequence) | *MDFQVQIFSFLLISASVIMSRG*QIVLTQSPAIMSASLGERVTMTC**TASS SVSSSYFH**WYQQKPGSSPKLWIY**STSNLAS**GVPARFSGSGSGTSY SLTVSSLEAEDAATYYC**HLYHRSPRT**FGGGTTLDIK | 31 |
| | *ATGGATTTTCAGGTGCAGATTTTCAGCTTCCTGCTAATCAGTGCCT CAGTCATAATGTCCAGAGGA*CAAATTGTTCTCACCCAGTCTCCAG CAATCATGTCTGCATCTCTAGGGGAACGGGTCACCATGACCTGC **ACTGCCAGCTCAAGTGTAAGTTCCAGTTATTTTCAC**TGGTACCAG CAGAAGCCAGGATCCTCACCCAAACTCTGGATTTAT**AGCACTTCC AACCTGGCTTCT**GGAGTCCCAGCTCGCTTCAGTGGCAGTGGGTC TGGGACCTCTTACTCTCTCACAGTCAGCAGTTTGGAGGCTGAGG ATGCTGCCACTTATTACTGC**CACCTGTATCATCGTTCCCCACGGA CG**TTCGGTGGAGGCACCACGCTGGATATCAAA | 32 |
| (In Table 3, the underlined regions represent CDR1, CDR2, and CDR3 in heavy and light chains, sequentially, with a signal sequence in italics) | | |

**1.2. Humanization of mouse anti-GITR clone**

Structural models of the antibody variable regions (V regions) were generated using Swiss PDB, and analyses were conducted to identify crucial "constraining" amino acids in the V regions that are potentially essential for antibody binding characteristics. Most of the residues included within the CDR (using both Kabat and Chothia definitions) and alongside numerous framework residues, were evaluated as significant.

Based on structural analysis, selection was made of human sequence fragments that can be utilized for creating humanized variants, and iTope-AI technology (ABZENA plc) was employed for in silico analysis of peptides binding to human MHC class II alleles. For areas outside and on the side of the CDR, a wide range of selected human sequence fragments was confirmed to be usable as components of the novel humanized variable region, whereas the choice of usable sequences narrows for the CDR region. Whenever possible, sequence fragments identified as significant non-human germline binders to human MHC class II were discarded. This reduced the set of fragments, and the combinations of these fragments were re-analyzed using the above methods to confirm that the junctions between the fragments did not contain potential T cell epitopes.

To generate variable region sequences that either lack (when possible) or have reduced significant T cell epitopes, the selected sequence fragments were assembled. Five heavy chain (VH1 to VH5) and five light chain (VL1 to VL5) sequences were designed for mammalian cell expression gene synthesis and pairing (Table 4).

An example of combinations of heavy and light chains to produce one chimeric antibody (VH0/VL0) and 25 humanized antibodies is illustratively represented in Table 4 below:

TABLE 4

| | | Heavy chain | | | | | |
|---|---|---|---|---|---|---|---|
| | | VH0 | VH1 | VH2 | VH3 | VH4 | VH5 |
| Light chain | VL0 | ○ | | | | | |
| | VL1 | | ○ | ○ | ○ | ○ | ○ |
| | VL2 | | ○ | ○ | ○ | ○ | ○ |
| | VL3 | | ○ | ○ | ○ | ○ | ○ |
| | VL4 | | ○ | ○ | ○ | ○ | ○ |
| | VL5 | | ○ | ○ | ○ | ○ | ○ |

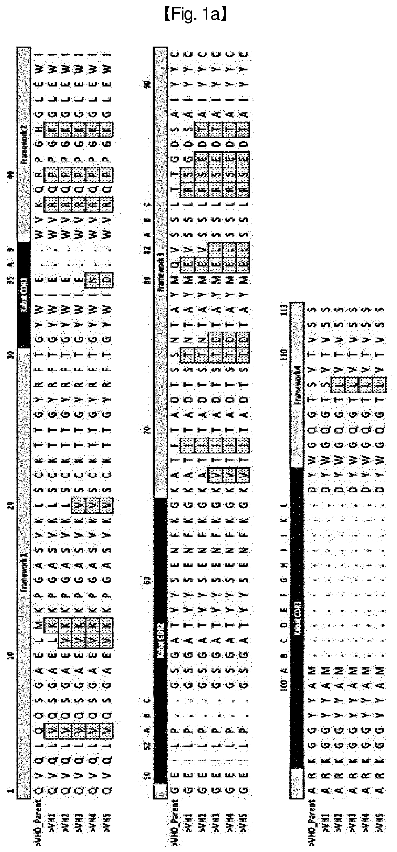

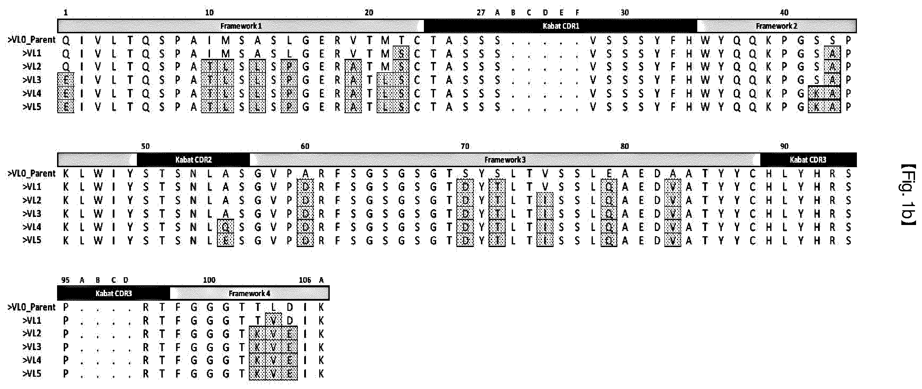

The sequences of the chimeric antibody (VH0xVL0) and humanized antibody, derived by applying the combination from Table 4 to the 51B clone from Table 3, are presented in Tables 5 and 6 and FIGS. 1a and 1b. In the sequences of Tables 5 and 6 and FIGS. 1a (heavy chain variable region) and 1b (light chain variable region), amino acid numbering and CDR regions were determined according to the Kabat definition, and amino acid residues that were changed from the CDR and parent sequence (VH0 or VL0) determined above are shaded in FIGS. 1a and 1b.

TABLE 5

| Heavy Chain (constant region: human IgG1) | | |
|---|---|---|
| | Amino acid sequence (N→C) | SEQ ID NO: |
| 51B_VH0 variable region | QVQLQQSGAELMKPGASVKLSCKTTGYRFTGYWIEWVKQRPG HGLEWIGEILPGSGATYYSENFKGKATFTADTSSNTAYMQVSSL TTGDSAIYYCARKGGYYAMDYWGQGTSVTVSS | 13 |
| 51B_VH1 variable region | QVQLVQSGAELKKPGASVKLSCKTTGYRFTGYWIEWVRQPPG KGLEWIGEILPGSGATYYSENFKGKATITADTSTNTAYMEVSSLR SGDSAIYYCARKGGYYAMDYWGQGTSVTVSS | 14 |

(continued)

| Heavy Chain (constant region: human IgG1) | | |
|---|---|---|
| | Amino acid sequence (N→C) | SEQ ID NO: |
| 51B_VH2 variable region | QVQLVQSGAEVKKPGASVKLSCKTTGYRFTGYWIEWVRQPPG KGLEWIGEILPGSGATYYSENFKGKATITADTSTNTAYMEVSSLR SEDTAIYYCARKGGYYAMDYWGQGTLVTVSS | 15 |
| 51B_VH3 variable region | QVQLVQSGAEVKKPGASVKVSCKTTGYRFTGYWIEWVRQPPG KGLEWIGEILPGSGATYYSENFKGKVTITADTSTDTAYMELSSLR SEDTAIYYCARKGGYYAMDYWGQGTLVTVSS | 16 |
| 51B_VH4 variable region | QVQLVQSGAEVKKPGASVKVSCKTTGYRFTGYWINWVRQPPG KGLEWIGEILPGSGATYYSENFKGKVTITADTSTDTAYMELSSLR SEDTAIYYCARKGGYYAMDYWGQGTLVTVSS | 17 |
| 51B_VH5 variable region | QVQLVQSGAEVKKPGASVKVSCKTTGYRFTGYWIDWVRQPPG KGLEWIGEILPGSGATYYSENFKGKVTITADTSTDTAYMELSSLR SEDTAIYYCARKGGYYAMDYWGQGTLVTVSS | 18 |
| Constant region (common) | ASTKGPSVFPLAPSSKSTSGGTAALGCLVKDYFPEPVTVSWNS GALTSGVHTFPAVLQSSGLYSLSSVVTVPSSSLGTQTYICNVNH KPSNTKVDKRVEPKSCDKTHTCPPCPAPELLGGPSVFLFPPKP KDTLMISRTPEVTCVVVDVSHEDPEVKFNWYVDGVEVHNAKTK PREEQYNSTYRVVSVLTVLHQDWLNGKEYKCKVSNKALPAPIE KTISKAKGQPREPQVYTLPPSREEMTKNQVSLTCLVKGFYPSDI AVEWESNGQPENNYKTTPPVLDSDGSFFLYSKLTVDKSRWQQ GNVFSCSVMHEALHNHYTQKSLSLSPGK | 25 |

TABLE 6

| Light Chain (constant region: kappa) | | |
|---|---|---|
| | Amino acid sequence (N→C) | SEQ ID NO: |
| 51B_VL0variable region | QIVLTQSPAIMSASLGERVTMTCTASSSVSSSYFHWYQQKPGS SPKLWIYSTSNLASGVPARFSGSGSGTSYSLTVSSLEAEDAATY YCHLYHRSPRTFGGGTTLDIK | 19 |
| 51B_VL1 variable region | QIVLTQSPAIMSASLGERVTMSCTASSSVSSSYFHWYQQKPGS APKLWIYSTSNLASGVPDRFSGSGSGTDYTLTVSSLQAEDVATY YCHLYHRSPRTFGGGTTVDIK | 20 |

(continued)

| | Light Chain (constant region: kappa) | |
|---|---|---|
| | Amino acid sequence (N→C) | SEQ ID NO: |
| 51B_VL2 variable region | QIVLTQSPATLSLSPGERATMSCTASSSVSSSYFHWYQQKPGS APKLWIYSTSNLASGVPDRFSGSGSGTDYTLTISSLQAEDVATY YCHLYHRSPRTFGGGTKVEIK | 21 |
| 51B_VL3 variable region | EIVLTQSPATLSLSPGERATLSCTASSSVSSSYFHWYQQKPGSA PKLWIYSTSNLASGVPDRFSGSGSGTDYTLTISSLQAEDVATYY CHLYHRSPRTFGGGTKVEIK | 22 |
| 51B_VL4 variable region | EIVLTQSPATLSLSPGERATLSCTASSSVSSSYFHWYQQKPGKA PKLWIYSTSNLQSGVPDRFSGSGSGTDYTLTISSLQAEDVATYY CHLYHRSPRTFGGGTKVEIK | 23 |
| 51B_VL5 variable region | EIVLTQSPATLSLSPGERATLSCTASSSVSSSYFHWYQQKPGKA PKLWIYSTSNLESGVPDRFSGSGSGTDYTLTISSLQAEDVATYY CHLYHRSPRTFGGGTKVEIK | 24 |
| Constant region (common) | RTVAAPSVFIFPPSDEQLKSGTASVVCLLNNFYPREAKVQWKVD NALQSGNSQESVTEQDSKDSTYSLSSTLTLSKADYEKHKVYAC EVTHQGLSSPVTKSFNRGEC | 26 |

In the following Examples, the same light chain clone may be denoted as either VL or Vk. For instance, VL0, VL1, VL2, VL3, VL4, and VL5 refer to the same clones as Vk0, Vk1, Vk2, Vk3, Vk4, and Vk5, respectively.

Among the antibodies produced, an Fc-engineered variant was created by introducing the N298A point mutation into the Fc region of the heavy chain of the 51B VH0/VL0 chimeric antibody. The sequences of the heavy and light chains of the antibody containing the Fc-engineered variant are listed in Table 7.

TABLE 7

| | Amino acid sequence (N→C) | SEQ ID NO: |
|---|---|---|
| 51B chimeric N298A_ VH (heavy chain) | QVQLQQSGAELMKPGASVKLSCKTTGYRFTGYWIEWVKQ RPGHGLEWIGEILPGSGATYYSENFKGKATFTADTSSNTA YMQVSSLTTGDSAIYYCARKGGYYAMDYWGQGTSVTVSS ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKRVEPKSCDKTHTCPPCPAPELLGG PSVFLFPPKPKDTLMISRTPEVTCVVVDVSHEDPEVKFNW YVDGVEVHNAKTKPREEQY**A**STYRVVSVLTVLHQDWLNG KEYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHY TQKSLSLSPGK | 27 |
| 51B chimeric N298A_ VL (light chain) | QIVLTQSPAIMSASLGERVTMTCTASSSVSSSYFHWYQQK PGSSPKLWIYSTSNLASGVPARFSGSGSGTSYSLTVSSLE AEDAATYYCHLYHRSPRTFGGGTTLDIKRTVAAPSVFIFPP SDEQLKSGTASVVCLLNNFYPREAKVQWKVDNALQSGNS QESVTEQDSKDSTYSLSSTLTLSKADYEKHKVYACEVTHQ GLSSPVTKSFNRGEC | 28 |
| (In Table 7, variable regions are underlined and mutated amino acid residues in Fc regions are bolded) | | |

### 1.3. Transient expression of chimeric and humanized IgG1 antibodies

The coding DNA for the VH0/VL0 chimeric antibody, two reference antibody coding DNAs, and the coding DNAs for combinations of humanized heavy chain and light chain (a total of 25 humanized pairings) were transiently transfected into HEK293 EBNA adherent cells (LGC Standards, Teddington, UK) in 6-well plates using the PEI transfection method. After transfection, the cells were cultured for 7 days. Samples were collected, and antibody concentrations were measured on the Octet QK384 using Protein A biosensors (Molecular Devices, Wokingham, Berkshire, UK), based on human IgG1 antibody standards.

The results obtained are presented in Table 8, below:

TABLE 8

| | | Heavy Chain | | | | | |
|---|---|---|---|---|---|---|---|
| | | VH0 | VH1 | VH2 | VH3 | VH4 | VH5 |
| | VL0 | 7.4 | | - | - | - | - |
| | VL1 | | 8.5 | 11.8 | 8.9 | 9.0 | 8.3 |
| Light chain | VL2 | - | 6.0 | 6.2 | 6.4 | 6.3 | 5.6 |
| | VL3 | - | 9.7 | 7.9 | 8.6 | 9.2 | 7.5 |
| | VL4 | - | 3.8 | 4.7 | 4.5 | 4.0 | 5.1 |
| | VL5 | - | 4.0 | 5.3 | 5.9 | 5.5 | 5.2 |

Table 8 shows the IgG concentrations ($\mu$g/mL) in the supernatants after the expression of the chimeric antibody (VH0/VL0) and 25 combinations of humanized antibodies in HEK cells.

As shown in Table 8, all the tested antibodies were generally well-expressed.

**1.4. Single cycle kinetic analysis of chimeric and humanized anti-GITR antibodies**

To evaluate the binding of all selected variants (humanized antibodies) to the human GITR antigen and to select a leading humanized IgG antibody with an affinity closest to the chimeric (VH0/VL0) antibody, a single cycle kinetic analysis was performed on the supernatant of the transfected cell cultures. The kinetic experiments were conducted using the Biacore 8K running Biacore 8K Control software V3.0.12.15655 and Biacore Insight Evaluation software V3.0.12.15655 (Cytiva, Uppsala, Sweden) under conditions at 25°C.

HBS-P$_+$ (Cytiva, Uppsala, Sweden) supplemented with 1% BSA w/v (Sigma, Dorset, UK) (to reduce non-specific binding to the reference surface) was used as the running buffer and also for dilution of the ligand and analyte. The supernatant containing IgG was diluted in the running buffer to 1 $\mu$g/mL. At the beginning of each cycle, the dilution was loaded onto Fc2 of channels 1-8 of the Protein A sensor chip (Cytiva, Little Chalfont, UK). IgG antibodies were captured at a flow rate of 10 $\mu$l/min, ensuring that the immobilization level (RL) reached approximately 130RU. Then, the surface was stabilized.

To minimize potential mass transfer effects, recombinant human GITR (Cat No., 13643-H08H, Lot No. LC12JA2509, Sino Biological, Beijing, China) injected at a flow rate of 40 $\mu$l/min was used as the analyte to obtain single cycle kinetic data. The antigen was diluted in the running buffer in a concentration range from 0.6125 nM to 10 nM by doubling (5 points) and used without regeneration between each concentration. For each injection of the five increasing antigen concentrations, the association phases were monitored for 200 seconds, and after the last antigen injection, the single dissociation phase was measured for 600 seconds. The sensor chip surface was regenerated by a single injection of 10 mM glycine (pH 1.5).

The double-referenced sensorgrams were fit to the Langmuir (1:1) binding model, and the accuracy (closeness) of the data to the model was assessed using the Chi square value, which explains the deviation between experimental and fitted (observed and expected) curves. The fitting algorithm was designed to minimize the Chi square value.

The results obtained are summarized in Table 9.

TABLE 9

| Antibody | ka(1/Ms) | kd(1/s) | KD(M) | Relative KD | Rmax (RU) | Chi$^2$ (RU$^2$) |
|---|---|---|---|---|---|---|
| VH0/VL0 | 5.90E+05 | 3.34E-04 | **5.66E-10** | 1 | 22.7 | 0.061 |
| VH0/VL1 | 6.42E+05 | 3.38E-04 | **5.27E-10** | 0.9 | 26.6 | 0.078 |
| VH1/VL0 | 6.03E+05 | 6.54E-04 | **1.08E-09** | 1.9 | 21.4 | 0.101 |
| VH1/VL1 | 7.21E+05 | 9.49E-04 | **1.32E-09** | 2.3 | 10.4 | 0.069 |
| VH1/VL2 | 5.15E+05 | 5.95E-04 | **1.16E-09** | 2.0 | 23.2 | 0.065 |
| VH1/VL3 | 5.21E+05 | 5.41E-04 | **1.04E-09** | 1.8 | 24.7 | 0.102 |
| VH1/VL4 | 4.69E+05 | 6.16E-04 | **1.31E-09** | 2.3 | 20.6 | 0.036 |
| VH1/VL5 | 4.45E+05 | 6.28E-04 | **1.41E-09** | 2.5 | 20.3 | 0.041 |

(continued)

| Antibody | ka(1/Ms) | kd(1/s) | KD(M) | Relative KD | Rmax (RU) | Chi$^2$ (RU$^2$) |
|---|---|---|---|---|---|---|
| VH2/VL1 | 6.65E+05 | 7.34E-04 | **1.10E-09** | 1.9 | 17.9 | 0.064 |
| VH2/VL2 | 5.48E+05 | 5.96E-04 | **1.09E-09** | 1.9 | 25.7 | 0.073 |
| VH2/VL3 | 5.15E+05 | 6.30E-04 | **1.22E-09** | 2.2 | 28.7 | 0.053 |
| VH2/VL4 | 5.02E+05 | 5.77E-04 | **1.15E-09** | 2.0 | 32.3 | 0.109 |
| VH2/VL5 | 4.77E+05 | 6.35E-04 | **1.33E-09** | 2.3 | 24.8 | 0.063 |
| VH3/VL1 | 7.84E+05 | 5.43E-04 | **6.93E-10** | 1.2 | 33.4 | 0.182 |
| VH3/VL2 | 6.66E+05 | 4.91E-04 | **7.37E-10** | 1.3 | 25.9 | 0.081 |
| VH3/VL3 | 6.37E+05 | 5.04E-04 | **7.91E-10** | 1.4 | 31.3 | 0.091 |
| VH3/VL4 | 6.16E+05 | 4.67E-04 | **7.58E-10** | 1.3 | 29.9 | 0.095 |
| VH3/VL5 | 5.54E+05 | 5.07E-04 | **9.16E-10** | 1.6 | 26.6 | 0.073 |
| VH4/VL1 | 6.27E+05 | 6.60E-04 | **1.05E-09** | 1.9 | 23.6 | 0.092 |
| VH4/VL2 | 5.35E+05 | 5.61E-04 | **1.05E-09** | 1.9 | 33.3 | 0.136 |
| VH4/VL3 | 5.54E+05 | 5.66E-04 | **1.02E-09** | 1.8 | 31.2 | 0.104 |
| VH4/VL4 | 5.37E+05 | 5.30E-04 | **9.87E-10** | 1.7 | 38.8 | 0.162 |
| VH4/VL5 | 4.74E+05 | 5.99E-04 | **1.26E-09** | 2.2 | 25 | 0.082 |
| VH5/VL1 | 3.82E+05 | 7.42E-04 | **1.94E-09** | 3.4 | 21.1 | 0.082 |
| VH5/VL2 | 4.12E+05 | 9.61E-04 | **2.33E-09** | 4.1 | 17.8 | 0.072 |
| VH5/VL3 | 3.84E+05 | 7.40E-04 | **1.93E-09** | 3.4 | 33 | 0.156 |
| VH5/VL4 | 1.08E+06 | 1.49E-03 | **1.37E-09** | 2.4 | 18.7 | 0.095 |
| VH5/VL5 | 2.94E+05 | 9.75E-04 | **3.32E-09** | 5.9 | 24.8 | 0.096 |

Table 9 shows the single cycle kinetic parameters for binding to the human GITR antigen of the chimeric (VH0/VL0) and humanized variants, as measured using the Biacore 8K. The relative KD was calculated by dividing the KD of the humanized variants by the KD of the VH0/VL0 chimeric antibody analyzed in the same experiment.

As seen in Table 9, the KD values related to the binding of all tested humanized variants (antibodies) to human GITR were observed to be within 5.9 times compared to VH0/VL0. Considering the relative expression levels, percentage of humanness, MHC class II binding risk, and relative KD values (obtained from Biacore single cycle kinetics analysis on the supernatant), six humanized variants (VH3/VL1, VH3/VL3, VH3/VL4, VH3/VL5, VH4/VL3, and VH4/VL4) were selected for subsequent thermal stability analysis.

### 1.5. Thermal stability assessment

To obtain information on the thermal stability of the antibody from the temperature at which it transitions from its native state to a denatured state (unfolding), a thermal ramp stability experiment (Tm and Tagg) was performed. Such unfolding processes occur over a narrow temperature range, and the midpoint of such a transition is called the "melting temperature" or "Tm". Since the protein undergoes a conformational change at this point, the fluorescence of Sypro Range (which binds to the exposed hydrophobic regions of the protein) was measured to determine the protein's melting temperature.

The samples were diluted in PBS to a final test concentration of 0.5mg/ml, and Sypro™ Orange (160x Stock solution; Sigma-Aldrich) was added to a final concentration of 20x solution. Each sample mixture was loaded twice into UNi microcuvettes, 9 μL each. A thermal ramp from 15°C to 95°C was applied to the samples (with a ramp rate of 0.3°C/minute and excitation at 473 nm). The complete emission spectra were collected from 250 to 720 nm, and the area under the curve between 510 - 680 nm was used to calculate the inflection points of the transition curve (Tonset and Tm). Static light scattering (SLS) at 473 nm was monitored to detect protein aggregation, and the Tagg (aggregation onset) was calculated from the resulting SLS profile. Data analysis was performed using UNcle™ software version 4.0 (ABZENA).

Table 10 shows thermal stability values of humanized and chimeric antibodies, obtained using the UNcle biostability platform.

TABLE 10

| Antibody | Tm1(°C) | | Tonset(°C) | | Tagg(°C)(473nm) | |
|---|---|---|---|---|---|---|
| | Average | SD | Average | SD | Average | SD |
| VH0/VL0 | 66.2 | 0.06 | 60.5 | 0.13 | 65.3 | 0.15 |
| VH3/VL1 | 66.1 | 0.26 | 59 | 0.11 | 65.2 | 0.09 |
| VH3/VL3 | 69.3 | 0.2 | 59.4 | 0.59 | 69.2 | 0.11 |
| VH3/VL4 | 66.7 | 0.04 | 59.6 | 0.12 | 66 | 0.02 |
| VH3/VL5 | 66.2 | 0.19 | 59.2 | 0.55 | 65.8 | 0.21 |
| VH4/VL3 | 70 | 1.09 | 59.2 | 0.33 | 70 | 0.01 |
| VH4/VL4 | 67.1 | 0.33 | 59.1 | 0.48 | 67.5 | 0.13 |

As shown in Table 10, most humanized antibodies showed heat stability equivalent to or better than that of chimeric antibodies.

**1.6. Affinity measurement of humanized variants using multi-cycle kinetic analysis**

To measure the binding affinity of the chimeric antibody and the six leading humanized variants (VH3/VL1, VH3/VL3, VH3/VL4, VH3/VL5, VH4/VL3, and VH4/VL4) for the human GITR antigen, a multi-cycle kinetic analysis was performed on the purified protein (antibody). The kinetic experiments were conducted at 25°C using Biacore 8K Control software V3.0.12.15655 and Biacore Insight Evaluation software V3.0.12.15655 (Cytiva, Uppsala, Sweden).

HBS-EP+ (Cytiva, Uppsala, Sweden) supplemented with 1% BSA w/v (Sigma, Dorset, UK) was used as the running buffer and also for dilution of the ligand and analyte. The purified leading antibodies were diluted to 1 μg/mL in the running buffer, and at the start of each cycle, they were loaded onto Fc2 of channels 1-8 of the Protein A sensor chip (Cytiva, Little Chalfont, UK). The antibodies were captured at a flow rate of 10 μl/min until the immobilization level (RL) reached approximately 120 RU.

Afterward, the surface was stabilized.

To minimize potential mass transfer effects, recombinant human GITR (Sino Biological, Beijing, China) was used as the analyte and injected at a flow rate of 30 μl/min to obtain multi-cycle kinetic data. The antigen (GITR) was diluted in running buffer from a concentration range of 1.25 nM to 40 nM, with two-fold dilutions at six points. At each concentration, the association phases were monitored for 210 seconds, and the dissociation phase was measured for 1200 seconds. The sensor chip surface was regenerated between cycles with a single injection of 10mM glycine (pH 1.5). To ensure the stability of both the surface and the analyte over kinetic cycles, repeated blanks and repeated injections of two analyte concentrations were programmed into the kinetic run.

The results obtained for the chimeric antibody (VH0/VL0) and the six leading antibodies are shown in Table 11.

TABLE 11

| Antibody | ka(1/Ms) | kd(1/s) | KD(M) | Relative KD | Rmax (RU) | Chi$^2$ (RU$^2$) |
|---|---|---|---|---|---|---|
| VH0/VL0 | 5.52E+05 | 3.14E-04 | **5.70E-10** | 1.00 | 27.2 | 3.83E-02 |
| VH3/VL1 | 7.96E+05 | 4.82E-04 | **6.06E-10** | 1.06 | 25.1 | 2.59E-01 |
| VH3/VL3 | 6.63E+05 | 4.26E-04 | **6.43E-10** | 1.13 | 25.0 | 1.41E-01 |
| VH3/VL4 | 6.36E+05 | 4.30E-04 | **6.75E-10** | 1.19 | 24.8 | 1.42E-01 |
| VH3/VL5 | 5.86E+05 | 4.56E-04 | **7.78E-10** | 1.37 | 26.1 | 1.75E-01 |
| VH4/VL3 | 6.26E+05 | 5.07E-04 | **8.10E-10** | 1.42 | 18.3 | 1.34E-01 |

(continued)

| Antibody | ka(1/Ms) | kd(1/s) | KD(M) | Relative KD | Rmax (RU) | Chi$^2$ (RU$^2$) |
|---|---|---|---|---|---|---|
| VH4/VL4 | 5.90E+05 | 4.88E-04 | **8.29E-10** | 1.45 | 23.6 | 2.41E-01 |

Table 11 displays the binding parameters of the purified six humanized antibodies and the chimeric antibody (VH0/VL0) to the human GITR antigen, measured using Biacore 8K.

As indicated in Table 11, all the tested antibodies exhibited excellent binding to human GITR, and the humanized antibodies all showed a high binding capacity within 1.5 times that of the VH0/VL0 chimeric antibody.

### 1.7. Epitope Mapping

To determine the epitope of the GITR/51B complex at high resolution, the protein complex was incubated with deuterated cross-linkers, followed by multi-enzymatic cleavage. The cross-linked peptides, once enriched, were then analyzed using a high-resolution mass spectrometer (nLC-Q-Exactive MS). The generated data was analyzed using XQuest and Stavrox software.

The analysis was performed using nLC chromatography in conjunction with Q-Exactive mass spectrometry.

Mixtures of hGITR (including His tag; Cat. GIR-H5228, Acrobiosystems) and the 51B antibody were prepared at the following concentrations:

TABLE 12

| Mixtures | GITR | | Antibodies | | GITR/ Antibodies | |
|---|---|---|---|---|---|---|
| | Volume | Conc. | Volume | Conc. | Volume | Conc. |
| GITR/51B | 10µl | 2 µM | 10µl | 1.1 µM | 20µl | 1 µM/0.55µM |

The 20 µL GITR/51B mixture, as prepared above, was mixed with 2 µL of DSS d0/d12 (2mg/mL, DMF) and incubated at room temperature for 180 minutes. After incubation, 1 µL of ammonium bicarbonate (final concentration 20 mM) was added to terminate the reaction, and it was then incubated at room temperature for an additional hour. The solution was then dried using a speedvac, followed by suspension in 8M urea in water (20µL). After mixing, 2 µl of DTT (500mM) was added. This mixture was incubated at 37°C for an hour. Subsequently, 2 µl of iodoacetamide (1M) was added, and the mixture was incubated in the dark at room temperature for another hour. After incubation, 80 µl of protein buffer was added. As for the protein buffer, the trypsin buffer contained 50 mM Ambic pH 8.5 and 5% acetonitrile; the chymotrypsin buffer contained 100 mM Tris HCl and 10mM CaCl$_2$ pH 7.8; the ASP-N buffer contained 50 mM phosphate buffer pH 7.8; the elastase buffer contained 50 mM Tris HCl pH 8.0; and the thermolysin buffer contained 50 mM Tris HCl and 0.5mM CaCl$_2$ pH 9.0.

The protease reactions were performed as follows:

### - Trypsin Proteolysis

One hundred µl of the prepared reduced/alkyled GITR/51B mixture was mixed at a ratio of 1/100 with 0.7 µl of trypsin (Promega). The protein digestion mixture was incubated overnight at 37°C.

### - Chymotrypsin Proteolysis

One hundred µl of the prepared reduced/alkyled GITR/51B mixture was mixed at a ratio of to 1/200 with 0.5µl of chymotrypsin (Promega). The protein digestion mixture was incubated overnight at 25°C.

### - ASP-N Proteolysis

One hundred µl of the prepared reduced/alkyled GITR/51B mixture was mixed at a ratio of 1/200 with 0.5µl of ASP-N (Promega). The protein digestion mixture was incubated overnight at 37°C.

### - Elastase Proteolysis

One hundred µl of the prepared reduced/alkyled GITR/51B mixture was mixed at a ratio of 1/100 with 0.7µl of elastase (Promega). The protein digestion mixture was incubated overnight at 37°C.

**- Thermolysin Proteolysis**

One hundred µl of the prepared reduced/alkyled GITR/51B mixture of 100µl was mixed at a ratio of 1/50 with 1.4 µl of thermolysin (Promega). The protein digestion mixture was incubated overnight at 70°C.

After the degradation, formic acid was added to a final concentration of 1% (v/v).

Cross-linked peptides were analyzed using Xquest version 2.0.

After trypsin, chymotrypsin, ASP-N, elastase, and thermolysin proteolysis (with deuterated d0d12) of the GITR/51B protein complex, 12 cross-linked peptides between GITR and 51B were detected through nLC-orbitrap MS/MS analysis.

As a result of the analysis, on the GITR amino acid sequence (amino acids 26-241 of SEQ ID NO: 33), amino acids 92S (the 92nd residue Ser(S) from the 26th amino acid of SEQ ID NO: 33, hereinafter construed in the same manner), 96S, 107T, 110T, 116T, 122K, and 132S were confirmed to be specifically cross-linked with the 51B antibody, and the following amino acid sequences were confirmed as the epitope on GITR:

    1. Residues 92-96 (SGTFS, SEQ ID NO: 34)
    2. Residues 107-116 (TDCTQFGFLT, SEQ ID NO: 35)
    3. Residues 122-132 (KTHNAVCVPGS, SEQ ID NO: 36)

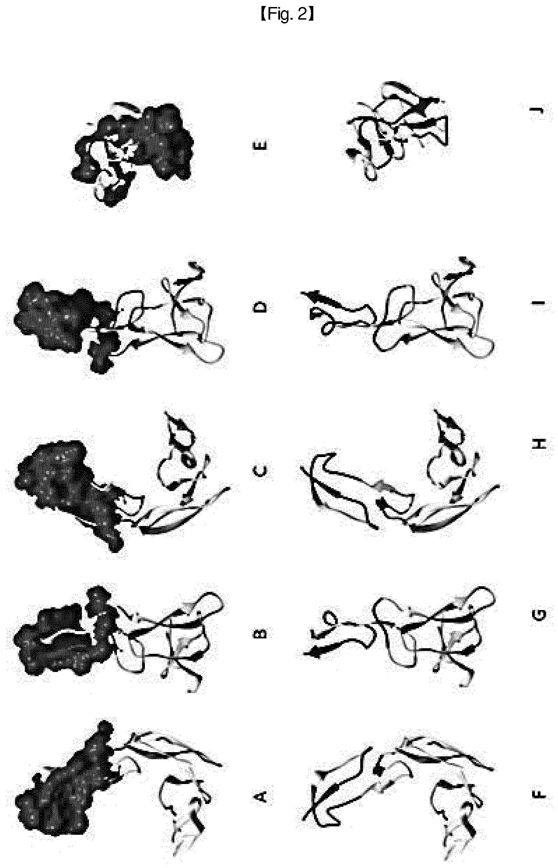

The identified epitope regions on GITR are depicted in FIG. 2. FIG. 2 illustrates the interaction site between GITR and 51B. The epitope region in the GITR PDB structure 7KHD is expressed in dark shading. The dark-shaded amino acids in the GITR protein structure correspond to sequences 92-96 (SGTPS, SEQ ID NO: 34), 107-116 (TDCTQFGFLT, SEQ ID NO: 35), and 122-132 (KTHNAVCVPGS, SEQ ID NO: 36) of the GITR sequence. In FIG 2, A, B, C, D, and E represent a front view (A), a rear view (B), a side view 1 (C), a side view 2 (D), and a plan view (E) of the ribbon/surface representation, while F, G, H, I, and J represent a front view (F), a rear view (G), a side view 1 (H), a side view 2 (I), and a plan view (J) of the ribbon representation, respectively.

**1.8. Specificity and binding affinity of anti-GITR antibody for human GITR protein**

**1.8.1. Binding analysis of anti-GITR antibody to human GITR (hGITR) protein (by ELISA)**

The ELISA plate was coated with recombinant human GITR protein (in PBS) at a concentration of 1 µg/ml and incubated overnight at 4°C. The plate was then blocked with blocking buffer (3% skim milk in PBS) for 1 hour, and serially diluted anti-GITR 51B VH0/Vk0 (chimeric aGITR 51B mAb) and anti-GITR 51B VH3/Vk4 (humanized anti-GITR 51B mAb) antibodies (starting concentration 3µg /ml; 1:3 serial dilution) were added to the coated plate. After incubation, anti-mouse/human IgG-HRP antibody (Abcam) (diluted 1:10000) was added to each well. TMB solution was added to the plate, and the reaction was stopped with 1N HCl. The OD values were read at 450 nm using the GloMax® Explorer Full Loaded.

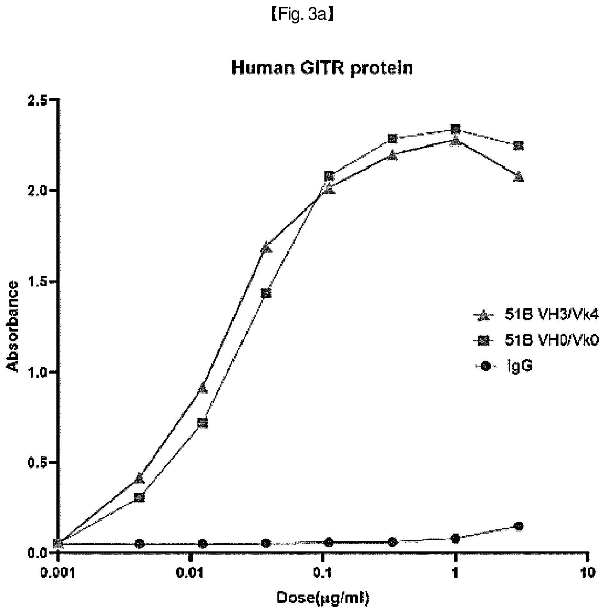

The obtained results are depicted in FIG. 3a. As indicated in FIG. 3a, the specificity of the tested anti-GITR 51B VH0/Vk0 chimeric antibody and anti-GITR 51B VH3/Vk4 humanized antibody binding to hGITR protein was confirmed. Both chimeric and humanized antibodies demonstrated significant binding to hGITR protein in a dose-dependent manner.

**1.8.2. Specificity and binding affinity of anti-GITR antibody for hGITR in human primary immune cells**

To measure the binding of the anti-GITR antibody to the GITR molecule expressed in human primary lymphocytes including T cells, NK cells, and NKT cells, the anti-GITR 51B VH0/Vk0 (chimeric mAb) and 51BVH3/Vk4 (humanized mAb) were each conjugated with Alexa Fluor®488 (AF488) using the APEX™ antibody labeling kit (Invitrogen) according to the manufacturer's instructions. Human peripheral blood mononuclear cells (PBMC) isolated from healthy donors were purchased from StemCell technologies and were cultured for 2 days in PRMI1640 media containing 10%(v/v) FBS and 400 U/ml human IL-2 in the presence of 1 µg/ml anti-CD3 monoclonal antibody (BD Biosciences) and 1 µg/ml anti-CD28 monoclonal antibody (BD Biosciences) under 5% $CO_2$ and 37°C conditions. During cultivation, IL-2 400 U/ml and IL-15 20 ng/ml were added every 2 or 3 days. After cultivation, the cells were stained with fluorophore-conjugated antibodies against T cell surface markers (CD3, CD4, and CD8) and NK or NKT cell surface markers (CD56) and various amounts (50, 250, 750, 1250, and 2500 ng/ml) of AF488-labeled anti-GITR antibody. The cells were analyzed with a flow cytometer (CytoFLEX, Beckman Coulter), and the data was analyzed using FlowJo software (BD). Binding of the anti-GITR antibody to GITR was measured in total T cells (CD3+), CD4+ T cells (CD3+CD4+CD8-), CD8+ T cells (CD3+CD8+CD4-), CD56+ NK cells (CD3-CD56+), and NKT cells (CD3+CD56+).

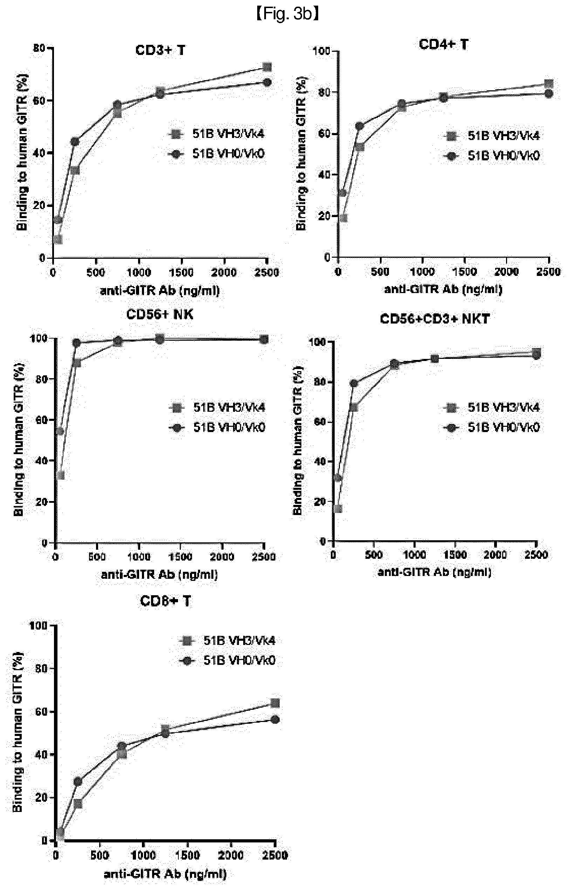

The obtained results are depicted in FIG. 3b. Both the chimeric and humanized anti-GITR 51B antibodies were confirmed to bind to primary T cells, NK, and NKT cells in a dose-dependent manner. The results demonstrate the specificity of the anti-GITR 51B antibody for the GITR protein expressed in immune cells.

## EXAMPLE 2. Cell-Based Reporter Assay for Biological Activity of Anti-GITR Antibody (Anti-GITR AgonistAactivity)

The Promega GITR bioassay kit was purchased from Promega Corporation (Madison, WI, USA). One vial (0.5 mL) of GITR effector cells (Promega) was added to 9.5mL of assay buffer (99% RPMI, 1% FBS), and 50 $\mu$L of this cell suspension was transferred to 96-well white flat-bottom assay plates (Greiner Bio-one, Chonburi, Thailand). Using the assay buffer, a continuous dilution of the anti-GITR antibody was prepared starting from a concentration of 5$\mu$g/ml, and then further diluted with the assay buffer by 2.5 times to a final antibody concentration of 0.0032768 $\mu$g/ml. Twenty-five $\mu$L of the diluted anti-GITR antibody was added to the assay plates. After incubation for 6 hours at 37°C and 5% $CO_2$, the Bio-Glo Reagent was added, followed by incubation at ambient temperature for 10 minutes. Luminescence was measured using the Promega™ GloMax® Plate Reader. Graphs were plotted for RLU versus Log10[antibody] and fold induction versus Log10[antibody]. Fit curves and the $EC_{50}$ value of the antibody response were measured using curve fitting software (such as GraphPad Prism® software). Through the above assay, the level of GITR-mediated luminescence was measured after the addition of the active GITR antibody (test antibody), and the agonist activity of the test antibody was confirmed. For comparison, the anti-GITR 28F3 antibody (BMS; see WO 2015/187835), anti-GITR 110416 (R&D Systems; refer to "Heine et al. Oncoimmunology 2017 and Schmiedel et al, Molecular Therapy 2013") and a positive control anti-GITR antibody (Promega Cat.# K1171) were used.

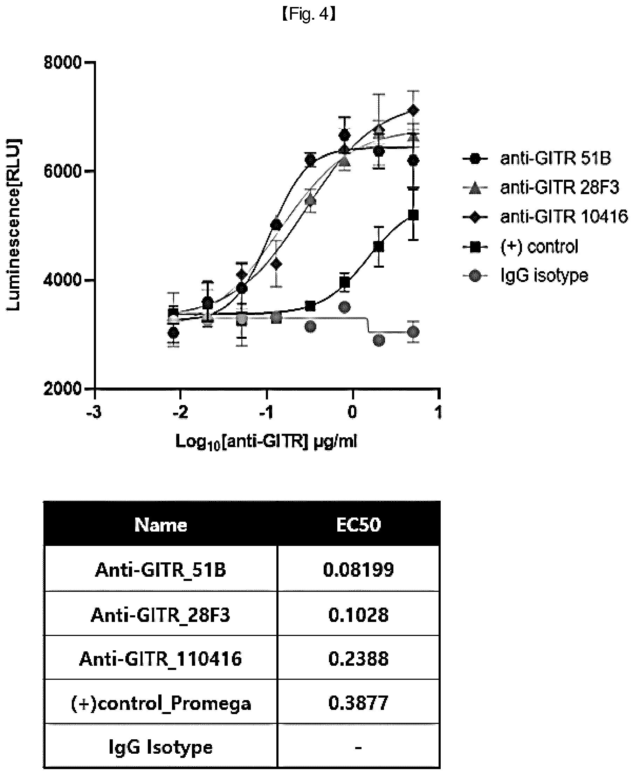

The results obtained are depicted in FIG. 4. It was confirmed that the signal transduction for T cell activation increased through the agonist activity of the anti-GITR antibody with the crosslinking of the anti-GITR 51B VH3/Vk5 antibody (indicated as anti-GITR 51B). This efficacy appeared to be significantly superior compared to the reference antibodies.

## EXAMPLE 3: Effect of Anti-GITR Antibody on Cytokine Production

### 3.1. Dose-dependent induction of anti-tumor cytokine production by anti-GITR antibody in human primary T cells

For cytokine production, human PBMCs (refer to Example 1.8.2) were cultured in 96-well round bottom plates with RPMI 1640 media supplemented with 10% heat-inactivated fetal bovine serum (FBS), 100 U/ml penicillin, and 100 $\mu$g/ml streptomycin at a concentration of $0.5 \times 10^6$ cells/well. In the presence of the anti-GITR antibody (chimeric antibody and six leading humanized antibodies, each at 10 $\mu$g/ml and 20 $\mu$g/ml), the cells were activated with 0.2 $\mu$g/ml of anti-CD3 monoclonal antibody and 1 $\mu$g/ml of anti-CD28 monoclonal antibody for 19 hours at 37°C and 5% $CO_2$. The anti-GITR antibody 110416 (R&D Systems) was used as a reference, and medium alone (no antibody added) was used as a negative control group. Brefeldin A was added to the co-stimulated cells (anti-CD3 and anti-CD28 antibodies) to block cytokine secretion. The cells were harvested, stained with Zombie Aqua fixable dead cell dye (Invitrogen), and incubated at room temperature for 20 minutes to measure cell viability. The cells were then stained with fluorophore-conjugated antibodies against the surface markers CD3, CD4, and CD8, all sourced from Biolegend, on ice for 30 minutes. After fixation and permeabilization of the cells with eBioscience™ Foxp3/Transcription Factor Fixation/Permeabilization Concentrate and Diluent kit (Thermofisher), intracellular cytokine staining was performed. Subsequently, the cells were stained in permeabilization buffer using fluorophore-conjugated antibodies to IL-2 and IFN-$\gamma$, both sourced from Biolegend, at room temperature for 30 minutes. The cells were then measured using a flow cytometer (CytoFLEX, Beckman Coulter), and the data were analyzed using FlowJo software (BD). The anti-GITR 110416 (R&D Systems) was used as a reference antibody.

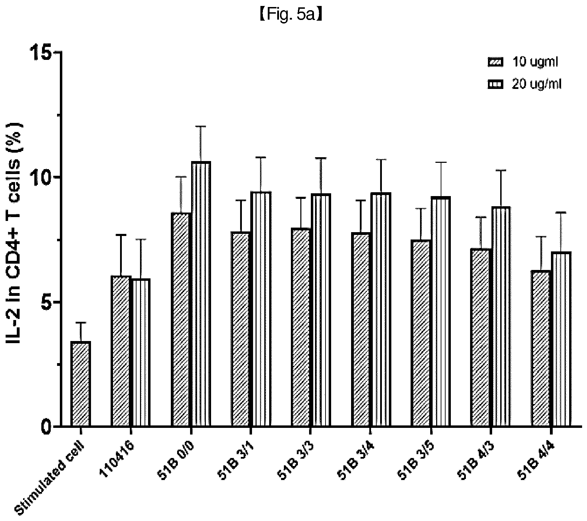

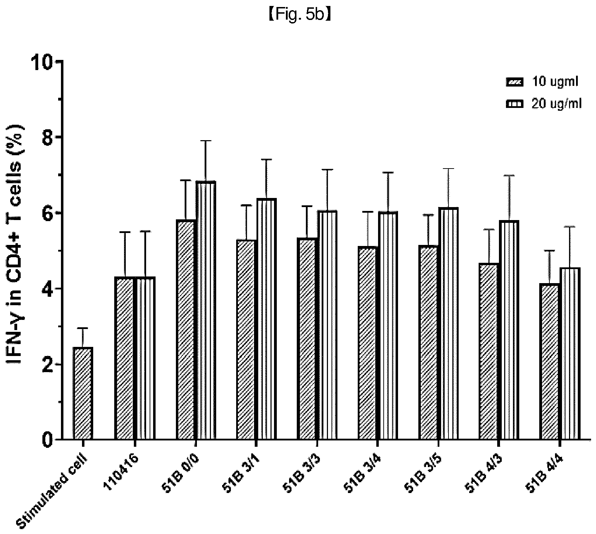

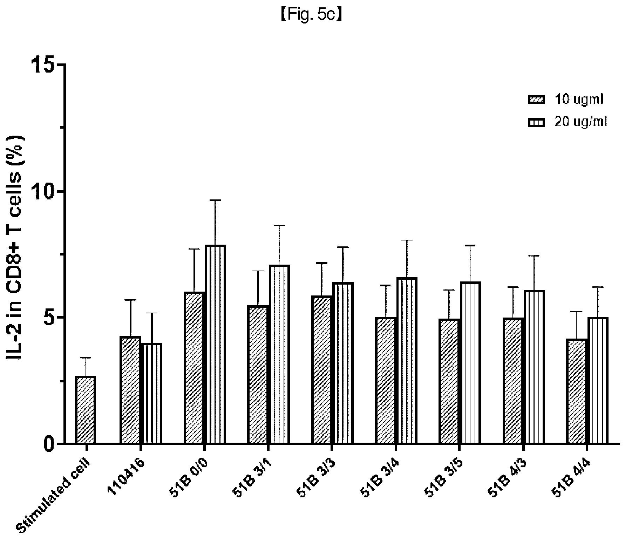

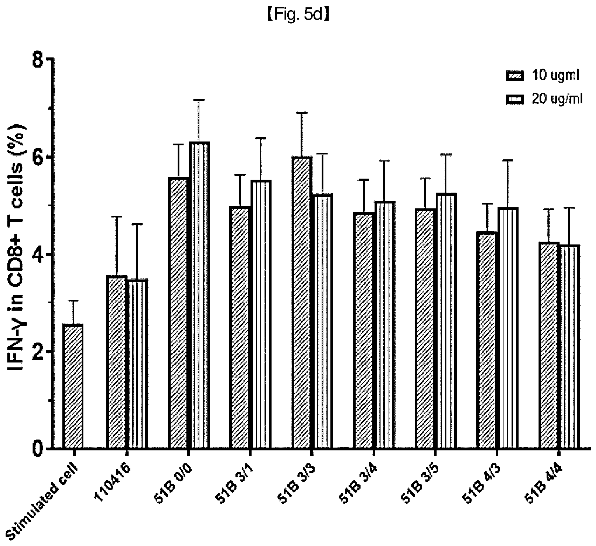

The results obtained are depicted in FIGS. 5a (IL-2 levels in CD4+ T cells), 5b (IFN-gamma levels in CD4+ T cells), 5c (IL-2 levels in CD8+ T cells), and 5d (IFN-gamma levels in CD8+ T cells). In FIGS 5a to 5d, the left of the paired bars for each antibody shows the results when the antibody concentration was 10 $\mu$g/ml, and the right shows the results when the antibody concentration was 20 $\mu$g/ml.

As shown in FIGS. 5a to 5d, all tested anti-GITR 51B antibodies (chimeric and humanized) upregulated the production of Th1/Tc1 cytokines in CD4+ and CD8+T cells through cross-linking with GITR on the surface of immune cells, thus enhancing the immune response. The levels of cytokines (IL-2 and IFN-gamma) during the treatment with anti-GITR 51B antibodies were significantly higher compared to the reference antibody (clone 110416, R&D System). These results demonstrate that all tested anti-GITR 51B antibodies enhance T cell function strongly and in a dose-dependent manner.

**3.2. Increased cytokine production by anti-GITR antibody in human T cells**

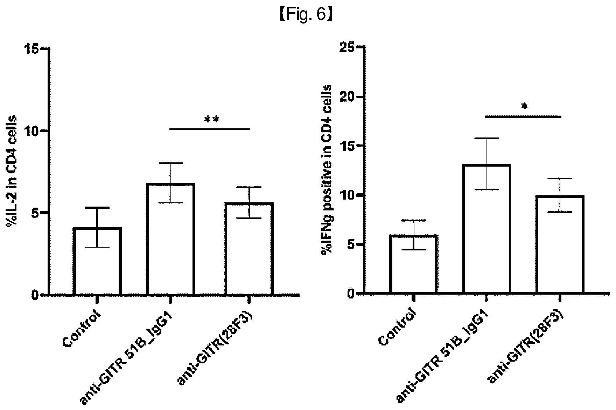

The cytokine production in human PBMCs (refer to Example 1.8.2) was measured. Cells (500,000 cells/reaction) were incubated with anti-CD3 monoclonal antibody (BD Biosciences, cat no. 555336; 0.2ug/ml) and anti-CD28 monoclonal antibody (BD Biosciences, cat no 555725; 1ug/ml) in the presence of the humanized anti-GITR 51B VH3/Vk4-IgG1 antibody (test antibody, 10 ug/ml). The anti-GITR antibody 28F3 (BMS) introduced as a reference antibody in the foregoing was used at the same concentration (10 ug/ml). After incubation, the cells were spun down at 400xg for 10 minutes at 4°C, and the cytokine levels within CD4+ T cells were measured. To this end, the intracellular cytokines were stained as follows: the cells were stained with the Zombie Aqua fixable dead cell dye solution (Invitrogen) and marked with fluorophore-conjugated antibodies against CD4+ and CD8+ T cell surface markers for 30 minutes on ice (using anti-CD3 antibody (Biolegend), anti-CD4 antibody (Biolegend), and anti-CD8 antibody (Biolegend) to stain the surfaces of CD4+ and CD8+ T cells). The cells were fixed and permeabilized using the eBioscience™ Foxp3/Transcription Factor Fixation/Permeabilization Concentrate and Diluent kit (ThermoFisher) according to the manufacturer's instructions. These cells were then stained with fluorophore-conjugated antibodies against intracellular IL-2 and IFNγ. The cells were analyzed using a flow cytometer (CytoFLEX, Beckman Coulter), and the data were analyzed with FlowJo software (BD).
The obtained results are depicted in FIG. 6. As shown in FIG. 6, the anti-GITR 51BVH3/Vk4-IgG1 antibody upregulated Th1/Tc1 cytokine production in CD4+ T cells, which can enhance the immune response. Also, the cytokine production level of the anti-GITR 51BVH3/Vk4-IgG1 antibody was higher compared to the reference antibody 28F3. These results suggest that the anti-GITR 51BVH3/Vk5-IgG1 antibody significantly enhances effector T cell function more powerfully than the reference antibody 28F3.

**EXAMPLE 4. Proliferative Effects of Anti-GITR Antibody on Immune Cells**

**4.1. Enhancement of NK cell proliferation by GITR Crosslinking in response to IL-15**

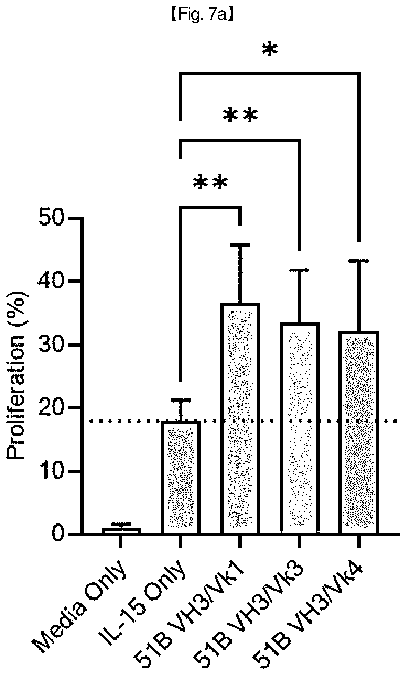

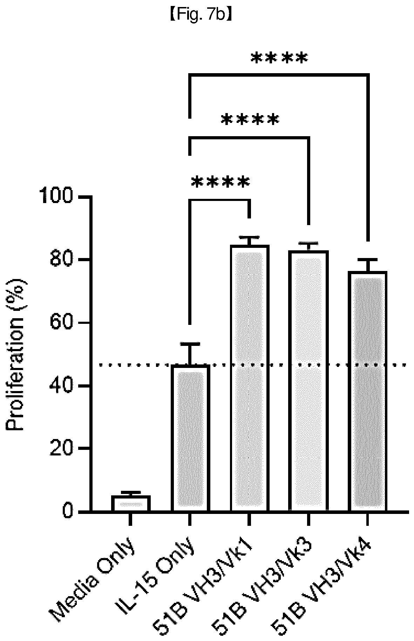

ELISA plates were coated with 10 μg/ml of humanized anti-GITR 51B antibodies (VH3/Vk1, VH3/Vk3, VH3/Vk4). NK cells were isolated from human PBMCs (refer to Example 1.8.2) using an NK isolation kit (Miltenyi Biotec) according to the manufacturer's instructions and stained with cell tracker violet (BioLegend) at 37°C for 20 minutes. The separated NK cells (refer to FIG. 7a) or PBMCs containing NK cells (refer to FIG. 7b, 500,000 cells/reaction) were added to the plates coated with the anti-GITR antibody and cultured with IL-15 (5 ng/ml) for 6 days. After culturing, the cells were stained with live/dead reagent (ThermoFisher LIVE/DEAD™ Fixable Near-IR Dead Cell Stain Kit), CD3 (Biolegend), and CD56 (Biolegend) antibodies, and then analyzed using a flow cytometer (CytoFlex, Beckman Coulter). Subsequently, the data were analyzed with FlowJo software (BD).
The results obtained are depicted in FIG. 7a (for separated NK cells) and 7b (for PBMCs containing NK cells). When GITR is crosslinked, IL-15-induced proliferation increases in both separated NK cells (A) and NK cells within PBMCs (B). All tested anti-GITR antibodies enhanced NK cell proliferation in human primary T cells. These results demonstrate that the anti-GITR antibodies have enhanced immune functions through cross-linking with GITR, suggesting potential anti-tumor activity.

**4.2. Enhancement of CD8+ T cell proliferation by anti-GITR antibody**

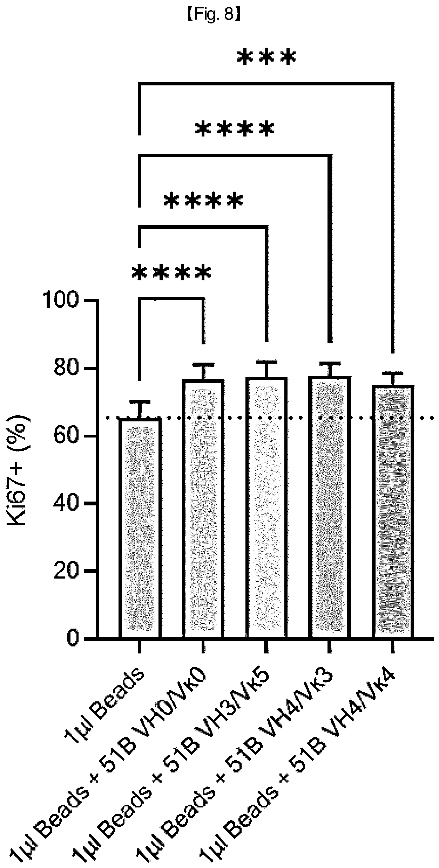

CD8+ T cells, Treg cells, and NK cells were isolated from human PBMCs (refer to Example 1.8.2) using Miltenyi kits according to the manufacturer's instructions (Miltenyi kits (Miltenyi Biotec): CD8+ T Cell Isolation Kit, human (130-096-495), CD4+ CD25+ CD127-dim Treg cell isolation kit II (130-094-775), and NK Cell Isolation Kit, human (130-092-657)). Isolated Treg cells were counted through trypan blue exclusion with the aid of the LUNA-II™ Automated Cell Counter. Cells were seeded in a ratio of NK cells: CD8 cells: Treg cells = 4:4:1 and anti-GITR antibodies 51B VH0/Vk0, VH3/Vk5, VH4/Vk3, and VG4/Vk4 were each added to the wells at a concentration of 10 μg/mL. The prepared mixture of cells and anti-GITR antibodies was incubated for 96 hours with CD3/CD28 Dynabeads (ThermoFisher) in the presence of IL-2 (50ng/ml). After culturing, the cells were stained for CD3, CD8, CD4, and live/dead markers, then fixed and permeabilized at 4°C for 20 minutes using CytoFix/CytoPerm (BD n. 554722). Subsequently, intracellular staining was performed using a Ki67 antibody to detect Ki67, and the cells were analyzed using a flow cytometer (CytoFlex, Beckman Coulter).
The results obtained are depicted in FIG. 8. As depicted in FIG. 8, the anti-GITR antibody increased the proliferation of CD8+ T cells in human primary PBMCs, suggesting that human immune cell functions can be enhanced by the anti-GITR antibody.

**4.3. Immunomodulation of T Regulatory Cells (Treg) and NK Cells by Anti-GITR Antibody**

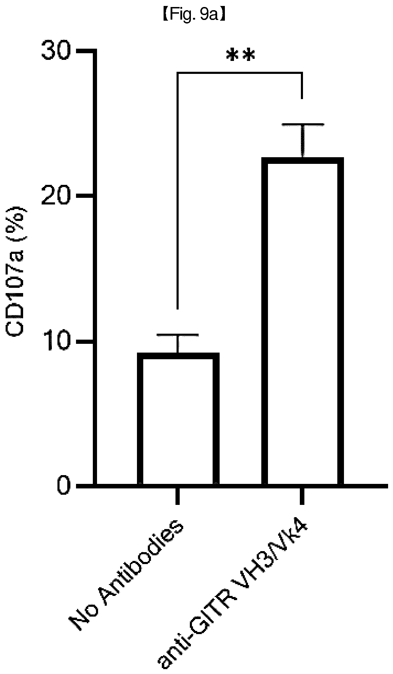

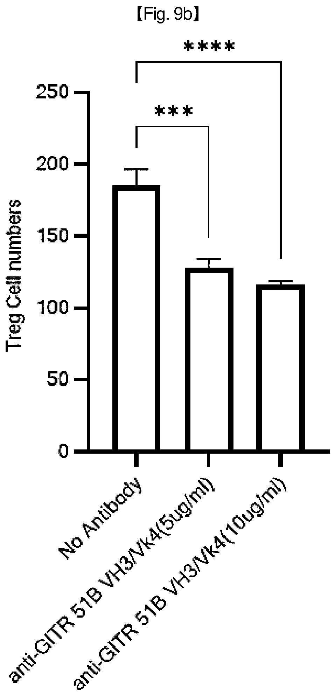

After incubating PBMCs (refer to Example 1.8.2) with the anti-GITR 51BVH3/Vk4 antibody, the activation of NK cells was measured by assessing CD107a in NK cells (refer to FIG. 9a), and the effect of the anti-GITR antibody on Treg depletion was evaluated (refer to FIG. 9b). More specifically, the anti-GITR 51B VH3/Vk4 antibody (at concentrations of 5 ug/ml and 10 ug/ml) was co-cultured with human PBMCs in the presence of CD3 and CD28 antibodies, and the frequency of Treg cells was evaluated through flow cytometry. The number of Treg cells was compared to the control group without an anti-GITR treatment.

The results obtained (at an antibody concentration of 10 ug/ml) are depicted in FIGS. 9a (levels of CD107a) and 9b (Treg depletion). Crosslinking of GITR through the anti-GITR 51BVH3/Vk4 antibody induces surface expression of CD107a on NK cells. Hence, the anti-GITR 51B VH3/Vk4 antibody significantly activates NK cells (refer to FIG. 9a). Additionally, co-culturing the anti-GITR antibody (anti-GITR 51B VH3/Vk4) with human PBMCs resulted in a decrease in the number of Treg cells (refer to FIG. 9b), suggesting that crosslinking of GITR with the anti-GITR antibody induces Treg removal by NK cells. Therefore, the anti-GITR antibody both induces NK cell activation and promotes the cytotoxicity of NK cells against Treg cells. Considering that in the tumor microenvironment, an increase in the number and function of Treg cells suppresses immune function with the resultant inhibition of anti-tumor effects, the tested anti-GITR antibody can potentially enhance anti-tumor effects by reducing the number of Treg cells.

**EXAMPLE 5. Anti-Tumor Effect of Anti-GITR Antibody**

Humanized variants of anti-GITR antibodies (anti-GITR 51B VH3/Vk4 and 51B VH3/Vk3 antibodies) were assayed for cytotoxicity against SKOV-3 ovarian cancer cells. In this regard, SKOV-3 cells (ATCC, $2.5 \times 10^4$ cells/well) and PBMCs (refer to Example 1.8.2) were co-cultured with the anti-GITR 51B antibodies (each at 10 $\mu$g/mL) so that the effector-to-target cell ratio (E:T) was 10:1. To activate T cells, Cytostim (Miltenyi) was added to each well. For comparison, tests were conducted in the same manner using the anti-GITR antibody 110416 (R&D System) as a reference antibody. Target cells were counted using IncuCyte native software.

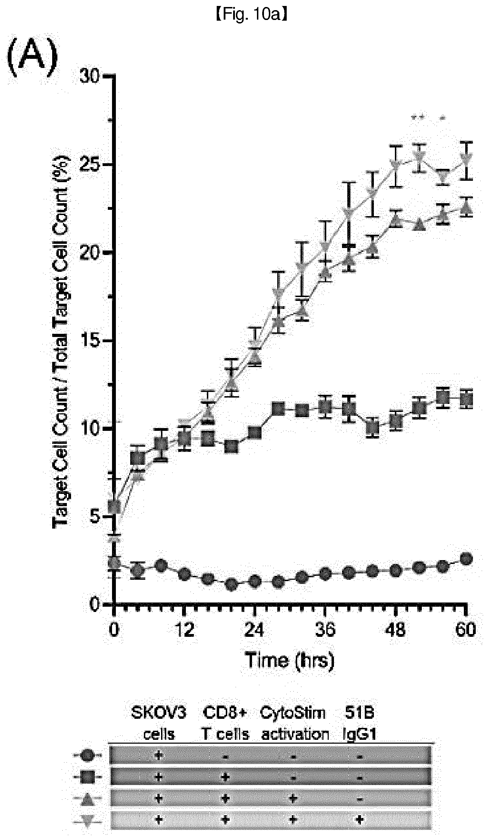

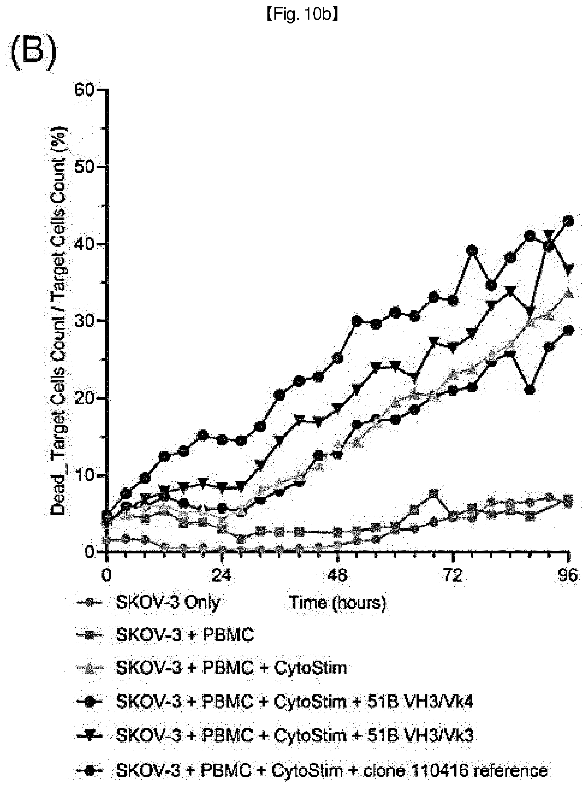

The results obtained (anti-GITR 51B VH3/Vk4 antibody) are depicted in FIGS. 10a and 10b. The tumoricidal effects of all the tested anti-GITR 51B antibodies were significantly higher than the control, the Cytostim-treated group, and the group treated with the reference antibody. The extent of ovarian cancer cell death increased due to the treatment with the test antibodies anti-GITR 51BVH3/Vk4 and anti-GITR 51BVH3/Vk3, implying that the anti-GITR 51B antibody (test antibody) exhibits strong anti-tumor activity. GITR crosslinking induced by the anti-GITR 51B antibody (test antibody) resulted in higher cytotoxicity against the SKOV3 cell line (ovarian cancer) compared to the reference antibody.

**EXAMPLE 6. Potent Anti-Tumor Cytokine Induction Effect of Anti-GITR Antibody in Human T Cells from Cancer Patients**

**6.1. Enhanced cytokine production in T cells from colorectal cancer (CRC) patients**

Since GITR-expressing immune cells cause functional impairment in cancer patients, examination was made to determine whether anti-GITR antibody treatment could induce anti-tumor effects by enhancing the immune response in cancer (CRC) patients.

To assess the effect of the anti-GITR antibody on cytokine production in cancer patients, human PBMCs (500,000 cells/reaction) derived from colorectal cancer (CRC) patients were cultured with anti-GITR 51 BVH3/Vk4 antibody (10 $\mu$g/mL) or an equivalent amount of the reference antibody (anti-GITR 28F3; BMS) in the presence of the anti-CD3 antibody (0.2 ug/ml, BD Biosciences) and anti-CD28 antibody (1 ug/ml, BD Biosciences). PBMCs from CRC patients were obtained from Cureline, Inc. Intracellular cytokine production was measured with reference to Example 3.

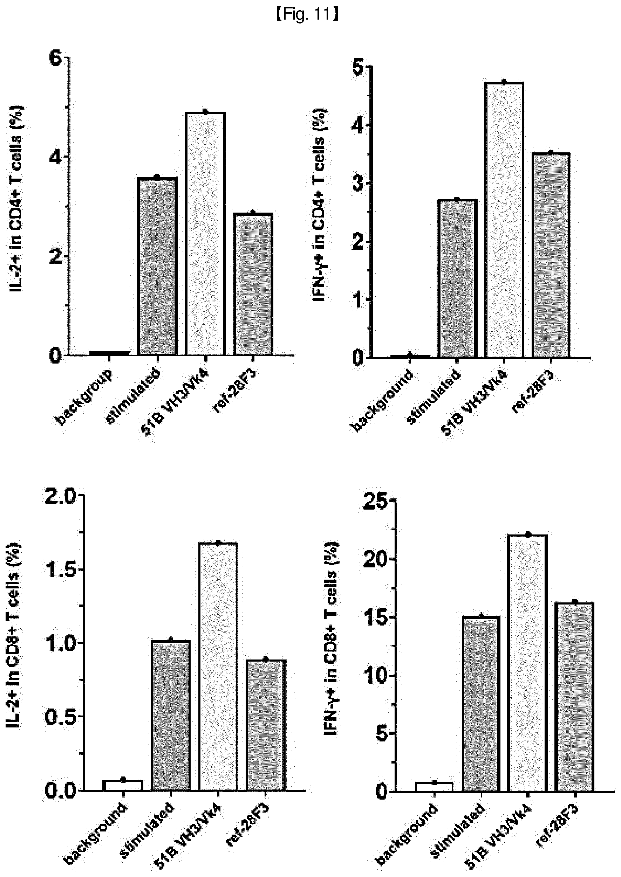

The results obtained are presented in FIG. 11 (Background: cells only (both untreated with CD3/CD28 antibodies and anti-GITR antibody), and Stimulated: treated with CD3/CD28 antibodies but untreated with anti-GITR antibody). As shown in FIG. 11, the test anti-GITR 51B VH3/Vk4 antibody (referred to as 51B VH3/Vk4) increased cytokine production in CD4+ and CD8+ T cells from CRC patient's PBMCs at a higher level than the reference antibody 28F3 (ref-28F3). IL-2 and IFN-$\gamma$ are cytokines with core functions in the immune system, enhancing the proliferation and tumoricidal activity of NK and T cells. Therefore, these results indicate that the test anti-GITR antibody exhibits superior anti-cancer efficacy in cancer (CRC) patients.

**6.2. Enhanced cytokine production in T cells from non-small-cell lung carcinoma (NSCLC) patients**

Since GITR-expressing immune cells cause functional impairment in cancer patients, examination was made to determine whether anti-GITR antibody treatment could induce anti-tumor effects by enhancing the immune response in cancer (NSCLC) patients.

To assess the effect of the anti-GITR antibody on cytokine production in cancer patients, human PBMCs (500,000 cells/reaction) derived from non-small-cell lung carcinoma (NSCLC) patients were cultured with anti-GITR 51 BVH3/Vk4 antibody (10 μg/mL) or an equivalent amount of the reference antibody (anti-GITR 28F3; BMS) in the presence of the anti-CD3 antibody (0.2 ug/ml, BD Biosciences) and anti-CD28 antibody (1 ug/ml, BD Biosciences). PBMCs from NSCLC patients were obtained from Cureline, Inc. Intracellular cytokine production was measured with reference to Example 3.

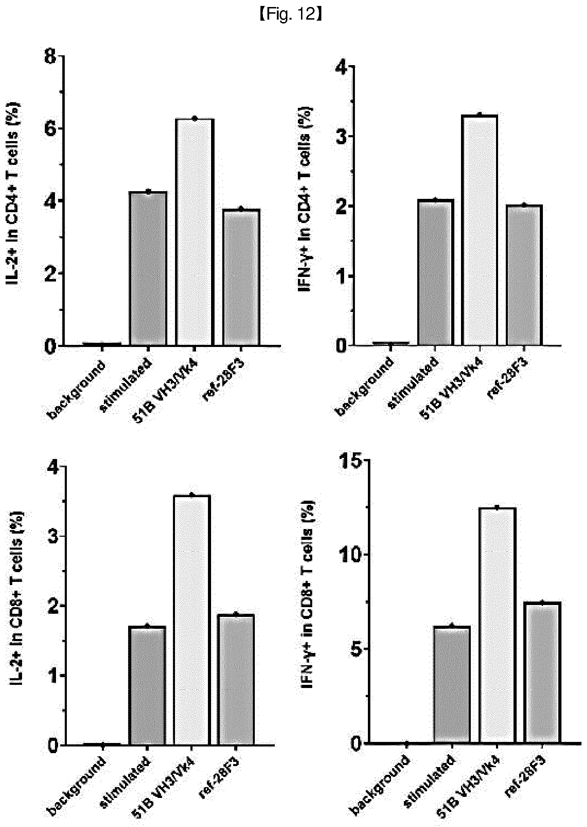

The results obtained are presented in FIG. 12 (Background: cells only (both untreated with CD3/CD28 antibodies and anti-GITR antibody), and Stimulated: treated with CD3/CD28 antibodies but untreated with anti-GITR antibody). As shown in FIG. 12, the test anti-GITR 51B VH3/Vk4 antibody (referred to as 51B VH3/Vk4) increased cytokine production in CD4+ and CD8+ T cells from NSCLC patient's PBMCs at a higher level than the reference antibody 28F3 (ref-28F3). IL-2 and IFN-γ are cytokines with core functions in the immune system, enhancing the proliferation and tumoricidal activity of NK and T cells. Therefore, these results indicate that the test anti-GITR antibody exhibits superior anti-cancer efficacy in cancer (NSCLC) patients.

### 6.3. Enhanced cytokine production in T cells from Hepatocellular carcinoma (HCC) patients

Since GITR-expressing immune cells cause functional impairment in cancer patients, examination was made to determine whether anti-GITR antibody treatment could induce anti-tumor effects by enhancing the immune response in cancer (HCC) patients.

To assess the effect of the anti-GITR antibody on cytokine production in cancer patients, human PBMCs (500,000 cells/reaction) derived from hepatocellular carcinoma (HCC) patients were cultured with anti-GITR 51 BVH3/Vk4 antibody (10 μg/mL) or an equivalent amount of the reference antibody (anti-GITR 28F3; BMS) in the presence of the anti-CD3 antibody (0.2 ug/ml, BD Biosciences) and anti-CD28 antibody (1 ug/ml, BD Biosciences). PBMCs from HCC patients were obtained from Cureline, Inc. Intracellular cytokine production was measured with reference to Example 3.

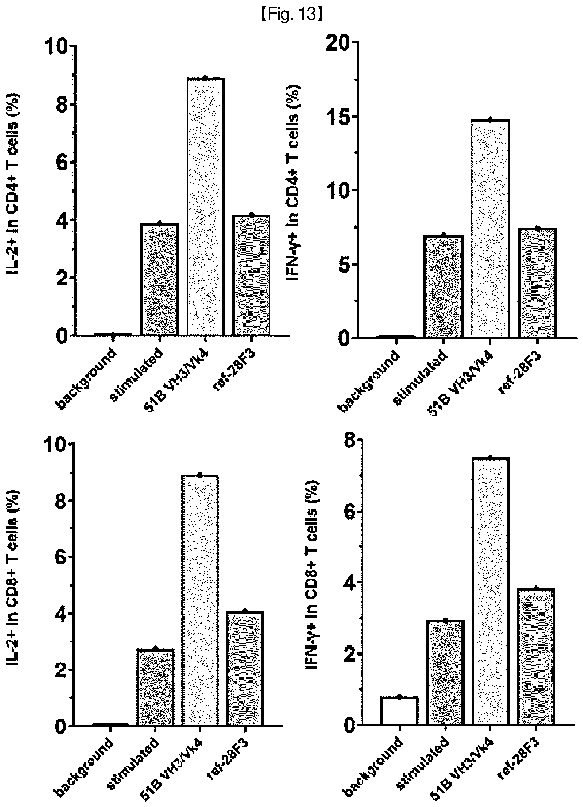

The results obtained are presented in FIG. 13 (Background: cells only (both untreated with CD3/CD28 antibodies and anti-GITR antibody), and Stimulated: treated with CD3/CD28 antibodies but untreated with anti-GITR antibody). As shown in FIG. 13, the test anti-GITR 51B VH3/Vk4 antibody (referred to as 51B VH3/Vk4) increased cytokine production in CD4+ and CD8+ T cells from HCC patient's PBMCs at a higher level than the reference antibody 28F3 (ref-28F3). IL-2 and IFN-γ are cytokines with core functions in the immune system, enhancing the proliferation and tumoricidal activity of NK and T cells. Therefore, these results indicate that the test anti-GITR antibody exhibits superior anti-cancer efficacy in cancer (HCC) patients.

As proven by the results, the test anti-GITR antibody induces potent anti-tumor cytokine production in human T cells from a wide range of cancer patients.

### EXAMPLE 7. Anti-Cancer Efficacy of Anti-GITR Antibody in MC38 Colon Carcinoma Model (in Vivo)

MC38 tumor cells (from Biocytogen) were maintained in vitro in DMEM supplemented with 10% (v/v) fetal bovine serum (FBS) and 4 ug/ml puromycin under conditions of 5% $CO_2$ and 37°C. Cells in the exponential growth phase were harvested, and counted with a cell counter before tumor inoculation.

For tumorigenesis, $5 \times 10^5$ tumor cells (in 0.1 mL of PBS solution) were subcutaneously inoculated into the right flank region of C57BL6 hGITR knock-in mice (from Jiangsu Biocytogen Co., Ltd.). Randomization was carried out when the average tumor size reached 100-150 mm$^3$. Each group was composed of eight mice. The date of randomization was recorded as day 0.

After tumor cell inoculation, the animals' morbidity, mortality, and weight gain/loss were monitored daily (post-randomization weight was measured three times per a week). Mortality and any observed clinical signs were meticulously recorded for each animal. Tumor volume was measured bi-dimensionally using a caliper thrice weekly post-randomization, and the volume was calculated in mm$^3$ using the formula:

$$V = (L \times W \times W) / 2$$

[V is the tumor volume (mm$^3$), L is the tumor length (the longest axis of the tumor), and W is the tumor width (the longest dimension perpendicular to L)].

Measurements of dosage and tumor and weight were performed in a Laminar Flow Cabinet, while the weight and tumor volume were measured using the StudyDirector™ software (version 3.1.399.19).

Treatment commenced immediately after grouping. The control group (Group 1) was treated with a vehicle (PBS solution), while Group 2 and Group 3 were treated with anti-GITR 51B VH3/Vk4 antibody produced in CHO cells and INCAGN1876 (from Biocytogen), respectively, at a dosage of 20 mg/kg (refer to Table 13).

TABLE 13

| Group | Treatment | Dose Level (mg/kg) | Dosing Volume (μL/g) | ROA | Dosing Frequency & Duration |
|---|---|---|---|---|---|
| 1 | Vehicle (PBS) | - | 10 | i.v. | BIW × 3 weeks |
| 2 | Anti-GITR 51B VH3/Vk4 | 20 | 10 | i.v. | BIW × 3 weeks |
| 3 | INCAGN1876 | 20 | 10 | i.v. | BIW × 3 weeks |
| BIW: Bi-weekly (twice a week) i.v.: intravenously administered | | | | | |

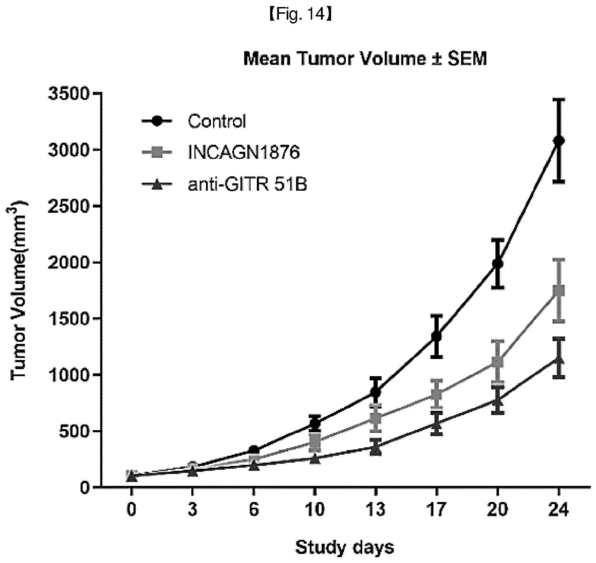

The tumor size (volume) measurements are depicted in FIG. 14. As illustrated in FIG. 14, the anti-GITR 51B VH3/Vk4 antibody (Group 2) exhibited a significant tumor growth inhibitory effect compared to Group 1 (the control group treated with PBS). For instance, on day 24, the tumor size in the test group treated with the anti-GITR 51B VH3/Vk4 antibody (Group 2) was significantly reduced compared to the control group (Group 1). Moreover, when compared with the group treated with the reference antibody (INCAGN1876) (Group 3), a remarkably higher tumor reduction efficacy was observed in the test group treated with the anti-GITR 51B VH3/Vk4 antibody (Group 2).

Furthermore, the tumor growth inhibition (ΔTGI) was calculated using the following equation:

$$\Delta TGI \ (Mean \ \% \ \Delta Inhibition) = ((mean(C)-mean(C0)) \ -(mean(T)-mean(T0)))$$

$$/(mean(C)-mean(C0)) * 100\%$$

$$[T - current \ group \ value \ / \ T0 - current \ group \ initial \ value \ / \ C - control \ group \ value$$

$$/ \ C0 - control \ group \ initial \ value]$$

[T: tumor size of the test group at the time of measurement,
T0: initial tumor size of the test group,
C: tumor size of the control group at the time of measurement,
C0: initial tumor size of the control group].

The tumor growth inhibition (ΔTGI) by the anti-GITR 51B VH3/Vk4 antibody was 64.8%, showing an enhanced inhibitory effect compared to the INCAGN1876 analog (ΔTGI=44.7%).

The test animals used in this Example tolerated the 20 mg/kg dose (i.v.) of the antibody, and no body weight loss (BWL) was observed during the study period.

**Statistical Analysis**

Statistical significance was evaluated using ordinary one-way ANOVA (paired and unpaired Student's tests using GraphPad Prism 9 software). Data were presented as mean ± SEM. **** $p<0.0001$ , *** $p<0.001$ , ** $p<0.01$ , * $p<0.05$, ns = not significant.

**Claims**

1. An anti-GITR antibody or an antigen-binding fragment thereof, wherein the anti-GITR antibody comprises:

   a polypeptide comprising the amino acid sequence of SEQ ID NO: 1 (CDR-H1),
   a polypeptide comprising the amino acid sequence of SEQ ID NO: 2 (CDR-H2),
   a polypeptide comprising the amino acid sequence of SEQ ID NO: 3 (CDR-H3),
   a polypeptide comprising the amino acid sequence of SEQ ID NO: 4 (CDR-L1),

a polypeptide comprising the amino acid sequence of SEQ ID NO: 5 (CDR-L2), and
a polypeptide comprising the amino acid sequence of SEQ ID NO: 6 (CDR-L3).

2. The anti-GITR antibody or antigen-binding fragment thereof according to claim 1, wherein the anti-GITR antibody comprises:

a polypeptide comprising the amino acid sequence of SEQ ID NO: 7, 8, or 9 (CDR-H1),
a polypeptide comprising the amino acid sequence of SEQ ID NO: 2 (CDR-H2),
a polypeptide comprising the amino acid sequence of SEQ ID NO: 3 (CDR-H3),
a polypeptide comprising the amino acid sequence of SEQ ID NO: 4 (CDR-L1),
a polypeptide comprising the amino acid sequence of SEQ ID NO: 10, 11, or 12 (CDR-L2), and
a polypeptide comprising the amino acid sequence of SEQ ID NO: 6 (CDR-L3).

3. The anti-GITR antibody or antigen-binding fragment thereof according to claim 1, wherein the anti-GITR antibody comprises:

(1) CDR-H1 comprising the amino acid sequence of SEQ ID NO: 7, CDR-H2 comprising the amino acid sequence of SEQ ID NO: 2, CDR-H3 comprising the amino acid sequence of SEQ ID NO: 3, CDR-L1 comprising the amino acid sequence of SEQ ID NO: 4, CDR-L2 comprising the amino acid sequence of SEQ ID NO: 10, and CDR-L3 comprising the amino acid sequence of SEQ ID NO: 6;
(2) CDR-H1 comprising the amino acid sequence of SEQ ID NO: 8, CDR-H2 comprising the amino acid sequence of SEQ ID NO: 2, CDR-H3 comprising the amino acid sequence of SEQ ID NO: 3, CDR-L1 comprising the amino acid sequence of SEQ ID NO: 4, CDR-L2 comprising the amino acid sequence of SEQ ID NO: 11, and CDR-L3 comprising the amino acid sequence of SEQ ID NO: 6; or
(3) CDR-H1 comprising the amino acid sequence of SEQ ID NO: 9, CDR-H2 comprising the amino acid sequence of SEQ ID NO: 2, CDR-H3 comprising the amino acid sequence of SEQ ID NO: 3, CDR-L1 comprising the amino acid sequence of SEQ ID NO: 4, CDR-L2 comprising the amino acid sequence of SEQ ID NO: 12, and CDR-L3 comprising the amino acid sequence of SEQ ID NO: 6.

4. The anti-GITR antibody or antigen-binding fragment thereof according to claim 1, wherein the anti-GITR antibody comprises:

a heavy variable region comprising the amino acid sequence of SEQ ID NO: 13, 14, 15, 16, 17, or 18; and
a light chain variable region comprising the amino acid sequence of SEQ ID NO: 19, 20, 21, 22, 23, or 24.

5. The anti-GITR antibody or antigen-binding fragment thereof according to claim 1, wherein the anti-GITR antibody or antigen-binding fragment thereof binds to at least one selected from the amino acids at positions 92-132 in the amino acid sequence of GITR protein.

6. The anti-GITR antibody or antigen-binding fragment thereof according to claim 1, wherein the anti-GITR antibody is an animal antibody, a chimeric antibody, or a humanized antibody.

7. The anti-GITR antibody or antigen-binding fragment thereof according to claim 1, wherein the antigen-binding fragment is scFv, scFv-Fc, (scFv)$_2$, Fab, Fab', or F(ab')$_2$ of the anti-GITR antibody.

8. A pharmaceutical composition for prevention or treatment of cancer, the composition comprising the anti-GITR antibody or antigen-binding fragment thereof according to any one of claims 1 to 7.

9. The pharmaceutical composition according to claim 8, wherein the cancer is solid cancer or hematological cancer.

10. A pharmaceutical composition for immunopotentiation, the composition comprising the anti-GITR antibody or antigen-binding fragment thereof according to any one of claims 1 to 7.

11. The pharmaceutical composition according to claim 10, wherein the composition has an activity selected from activating immune cells, inhibiting regulatory T cells, and increasing immune proteins.

12. A pharmaceutical composition for prevention or treatment of an immune-related disease, the composition comprising the anti-GITR antibody or antigen-binding fragment thereof according to any one of claims 1 to 7.

13. The pharmaceutical composition according to claim 12, wherein the immune-related disease is an infectious disease, an inflammatory disease, or an autoimmune disease.

【Fig. 1a】

【Fig. 1b】

【Fig. 2】

【Fig. 3a】

Human GITR protein

【Fig. 3b】

【Fig. 4】

| Name | EC50 |
|---|---|
| Anti-GITR_51B | 0.08199 |
| Anti-GITR_28F3 | 0.1028 |
| Anti-GITR_110416 | 0.2388 |
| (+)control_Promega | 0.3877 |
| IgG Isotype | - |

【Fig. 5a】

【Fig. 5b】

【Fig. 5c】

【Fig. 5d】

【Fig. 6】

【Fig. 7a】

【Fig. 7b】

【Fig. 8】

【Fig. 9a】

【Fig. 9b】

【Fig. 10a】

(A)

| | SKOV3 cells | CD8+ T cells | CytoStim activation | 51B IgG1 |
|---|---|---|---|---|
| ● | + | - | - | - |
| ■ | + | + | - | - |
| ▲ | + | + | + | - |
| ▼ | + | + | + | + |

【Fig. 10b】

(B)

SKOV-3 Only
SKOV-3 + PBMC
SKOV-3 + PBMC + CytoStim
SKOV-3 + PBMC + CytoStim + 51B VH3/Vk4
SKOV-3 + PBMC + CytoStim + 51B VH3/Vk3
SKOV-3 + PBMC + CytoStim + clone 110416 reference

【Fig. 11】

【Fig. 12】

【Fig. 13】

【Fig. 14】

**Mean Tumor Volume ± SEM**

## INTERNATIONAL SEARCH REPORT

International application No.

**PCT/KR2022/006697**

### A. CLASSIFICATION OF SUBJECT MATTER

**C07K 16/28**(2006.01)i; **A61P 35/00**(2006.01)i; **A61K 39/00**(2006.01)i

According to International Patent Classification (IPC) or to both national classification and IPC

### B. FIELDS SEARCHED

Minimum documentation searched (classification system followed by classification symbols)

C07K 16/28(2006.01); A61K 39/00(2006.01); A61K 39/395(2006.01); C12N 5/0783(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: GITR(glucocorticoid-induced TNFR-related protein), TNFRSF18, AITR, CD357, 항체(antibody), 암(cancer)

### C. DOCUMENTS CONSIDERED TO BE RELEVANT

| Category* | Citation of document, with indication, where appropriate, of the relevant passages | Relevant to claim No. |
|---|---|---|
| A | WO 2015-184099 A1 (4-ANTIBODY AG et al.) 03 December 2015 (2015-12-03)<br>See abstract; and claims 1-205. | 1-13 |
| A | KR 10-2019-0124753 A (RINAT NEUROSCIENCE CORP.) 05 November 2019 (2019-11-05)<br>See abstract; and claims 1-35. | 1-13 |
| A | KR 10-2019-0095298 A (POTENZA THERAPEUTICS, INC.) 14 August 2019 (2019-08-14)<br>See abstract; and claims 1-157. | 1-13 |
| A | WO 2017-068186 A1 (ABLYNX NV) 27 April 2017 (2017-04-27)<br>See abstract; and claims 1-85. | 1-13 |
| A | KR 10-2018-0127541 A (BRISTOL-MYERS SQUIBB COMPANY) 28 November 2018 (2018-11-28)<br>See abstract; and claims 1-68. | 1-13 |

☐ Further documents are listed in the continuation of Box C.    ☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **10 August 2022** | **10 August 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
|---|
| **PCT/KR2022/006697** |

---

**Box No. I    Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

---

Form PCT/ISA/210 (continuation of first sheet) (July 2019)

**INTERNATIONAL SEARCH REPORT**
Information on patent family members

| | International application No. |
|---|---|
| | **PCT/KR2022/006697** |

| Patent document cited in search report | | | Publication date (day/month/year) | Patent family member(s) | | | Publication date (day/month/year) |
|---|---|---|---|---|---|---|---|
| WO | 2015-184099 | A1 | 03 December 2015 | AR | 100640 | A1 | 19 October 2016 |
| | | | | AU | 2015-266958 | A1 | 08 December 2016 |
| | | | | AU | 2021-200582 | A1 | 04 March 2021 |
| | | | | CA | 2949998 | A1 | 03 December 2015 |
| | | | | CN | 108064242 | A | 22 May 2018 |
| | | | | DK | 3148579 | T3 | 08 March 2021 |
| | | | | EA | 201692458 | A1 | 30 June 2017 |
| | | | | EP | 3148579 | A1 | 05 April 2017 |
| | | | | EP | 3148579 | B1 | 16 December 2020 |
| | | | | EP | 3498295 | A1 | 19 June 2019 |
| | | | | ES | 2860751 | T3 | 05 October 2021 |
| | | | | HR | P20210364 | T1 | 30 April 2021 |
| | | | | HU | E053857 | T2 | 28 July 2021 |
| | | | | IL | 249092 | A | 31 January 2017 |
| | | | | JP | 2017-526615 | A | 14 September 2017 |
| | | | | JP | 2020-048584 | A | 02 April 2020 |
| | | | | JP | 6847666 | B2 | 24 March 2021 |
| | | | | KR | 10-2017-0020819 | A | 24 February 2017 |
| | | | | LT | 3148579 | T | 25 May 2021 |
| | | | | MA | 40041 | A | 05 April 2017 |
| | | | | MA | 40041 | B1 | 31 March 2021 |
| | | | | MA | 47849 | A | 29 January 2020 |
| | | | | MX | 2016015614 | A | 21 August 2017 |
| | | | | PH | 12016502345 | A1 | 13 February 2017 |
| | | | | PL | 3148579 | T3 | 19 July 2021 |
| | | | | RS | 61678 | B1 | 31 May 2021 |
| | | | | SG | 10201912986 | A | 27 February 2020 |
| | | | | SG | 11201609721 | A | 29 December 2016 |
| | | | | SI | 3148579 | T1 | 30 July 2021 |
| | | | | TW | 201607958 | A | 01 March 2016 |
| | | | | TW | 202132337 | A | 01 September 2021 |
| | | | | TW | I709573 | B | 11 November 2020 |
| | | | | US | 10155818 | B2 | 18 December 2018 |
| | | | | US | 10280226 | B2 | 07 May 2019 |
| | | | | US | 10577426 | B2 | 03 March 2020 |
| | | | | US | 10800849 | B2 | 13 October 2020 |
| | | | | US | 10829559 | B2 | 10 November 2020 |
| | | | | US | 2015-0368349 | A1 | 24 December 2015 |
| | | | | US | 2018-0244793 | A1 | 30 August 2018 |
| | | | | US | 2018-0355051 | A1 | 13 December 2018 |
| | | | | US | 2019-0010239 | A1 | 10 January 2019 |
| | | | | US | 2019-0062446 | A1 | 28 February 2019 |
| | | | | US | 2019-0309082 | A1 | 10 October 2019 |
| | | | | US | 2020-0339698 | A1 | 29 October 2020 |
| | | | | US | 2021-0070872 | A1 | 11 March 2021 |
| KR | 10-2019-0124753 | A | 05 November 2019 | AU | 2018-226646 | A1 | 19 September 2019 |
| | | | | AU | 2021-203126 | A1 | 10 June 2021 |
| | | | | BR | 112019017500 | A2 | 14 April 2020 |
| | | | | CA | 3054885 | A1 | 07 September 2018 |
| | | | | CN | 110431152 | A | 08 November 2019 |

Form PCT/ISA/210 (patent family annex) (July 2019)

**INTERNATIONAL SEARCH REPORT**
Information on patent family members

International application No.

**PCT/KR2022/006697**

| Patent document cited in search report | | | Publication date (day/month/year) | Patent family member(s) | | | Publication date (day/month/year) |
|---|---|---|---|---|---|---|---|
| | | | | CO | 2019009586 | A2 | 18 September 2019 |
| | | | | EP | 3589657 | A1 | 08 January 2020 |
| | | | | JP | 2020-510435 | A | 09 April 2020 |
| | | | | MX | 2019010458 | A | 20 January 2020 |
| | | | | PE | 20191487 | A1 | 18 October 2019 |
| | | | | PH | 12019502007 | A1 | 15 June 2020 |
| | | | | RU | 2019127550 | A | 05 April 2021 |
| | | | | SG | 11201907835 | A | 27 September 2019 |
| | | | | US | 11198735 | B2 | 14 December 2021 |
| | | | | US | 2020-0031947 | A1 | 30 January 2020 |
| | | | | WO | 2018-158658 | A1 | 07 September 2018 |
| KR | 10-2019-0095298 | A | 14 August 2019 | AR | 112904 | A1 | 08 January 2020 |
| | | | | AU | 2017-362721 | A1 | 23 May 2019 |
| | | | | BR | 112019010128 | A2 | 08 October 2019 |
| | | | | CA | 3042727 | A1 | 24 May 2018 |
| | | | | CN | 110248960 | A | 17 September 2019 |
| | | | | CO | 2019006424 | A2 | 20 August 2019 |
| | | | | EP | 3541845 | A1 | 25 September 2019 |
| | | | | IL | 266720 | A | 31 July 2019 |
| | | | | JP | 2019-537449 | A | 26 December 2019 |
| | | | | MA | 46844 | A | 25 September 2019 |
| | | | | MX | 2019005821 | A | 07 October 2019 |
| | | | | PH | 12019501108 | A1 | 29 July 2019 |
| | | | | RU | 2019118800 | A | 21 December 2020 |
| | | | | RU | 2019118800 | A3 | 23 March 2021 |
| | | | | TW | 201819416 | A | 01 June 2018 |
| | | | | US | 110988545 | B2 | 27 April 2021 |
| | | | | US | 2018-0208665 | A1 | 26 July 2018 |
| | | | | US | 2021-00269542 | A1 | 02 September 2021 |
| | | | | WO | 2018-094300 | A1 | 24 May 2018 |
| | | | | WO | 2018-094300 | A8 | 02 May 2019 |
| WO | 2017-068186 | A1 | 27 April 2017 | AU | 2016-341403 | A1 | 10 May 2018 |
| | | | | CA | 3002711 | A1 | 27 April 2017 |
| | | | | CN | 108473579 | A | 31 August 2018 |
| | | | | EP | 3365371 | A1 | 29 August 2018 |
| | | | | HK | 1258509 | A1 | 15 November 2019 |
| | | | | JP | 2018-537421 | A | 20 December 2018 |
| | | | | JP | 7084870 | B2 | 15 June 2022 |
| | | | | US | 11059899 | B1 | 13 July 2021 |
| | | | | WO | 2017-068186 | A9 | 22 June 2017 |
| KR | 10-2018-0127541 | A | 28 November 2018 | AR | 100744 | A1 | 26 October 2016 |
| | | | | AU | 2015-271709 | A1 | 08 December 2016 |
| | | | | AU | 2015-271709 | B2 | 26 November 2020 |
| | | | | BR | 112016028378 | A2 | 24 October 2017 |
| | | | | CA | 2951234 | A1 | 10 December 2015 |
| | | | | CL | 2016003136 | A1 | 08 September 2017 |
| | | | | CN | 106459203 | A | 22 February 2017 |
| | | | | CN | 106459203 | B | 03 August 2021 |
| | | | | DK | 3151921 | T3 | 02 December 2019 |
| | | | | EA | 037006 | B1 | 26 January 2021 |

Form PCT/ISA/210 (patent family annex) (July 2019)

INTERNATIONAL SEARCH REPORT
Information on patent family members

International application No.

**PCT/KR2022/006697**

| Patent document cited in search report | Publication date (day/month/year) | Patent family member(s) | | Publication date (day/month/year) |
|---|---|---|---|---|
| | | EA | 201692459 A1 | 28 April 2017 |
| | | EP | 3151921 A2 | 12 April 2017 |
| | | EP | 3151921 B1 | 28 August 2019 |
| | | EP | 3610924 A1 | 19 February 2020 |
| | | EP | 3610924 B1 | 03 November 2021 |
| | | EP | 3998079 A1 | 18 May 2022 |
| | | ES | 2755395 T3 | 22 April 2020 |
| | | HR | P20192098 T1 | 21 February 2020 |
| | | HU | E047385 T2 | 28 April 2020 |
| | | IL | 248945 A | 31 January 2017 |
| | | JP | 2017-523771 A | 24 August 2017 |
| | | JP | 2019-058181 A | 18 April 2019 |
| | | JP | 2021-119173 A | 12 August 2021 |
| | | JP | 6449338 B2 | 09 January 2019 |
| | | JP | 6878385 B2 | 26 May 2021 |
| | | KR | 10-1923326 B1 | 29 November 2018 |
| | | KR | 10-2017-0010438 A | 31 January 2017 |
| | | LT | 3151921 T | 10 December 2019 |
| | | MA | 39559 A1 | 31 August 2018 |
| | | MX | 2016015456 A | 23 February 2017 |
| | | PE | 20170441 A1 | 26 April 2017 |
| | | PH | 12016502267 A1 | 06 February 2017 |
| | | PT | 3151921 T | 21 November 2019 |
| | | RS | 59643 B1 | 31 January 2020 |
| | | SG | 10201810507 A | 28 December 2018 |
| | | SG | 11201610074 A | 29 December 2016 |
| | | SI | 3151921 T1 | 31 December 2019 |
| | | TW | 201613972 A | 16 April 2016 |
| | | TW | I695843 B | 11 June 2020 |
| | | US | 10465010 B2 | 05 November 2019 |
| | | US | 10501550 B2 | 10 December 2019 |
| | | US | 11084881 B2 | 10 August 2021 |
| | | US | 2015-0353637 A1 | 10 December 2015 |
| | | US | 2016-0145342 A1 | 26 May 2016 |
| | | US | 2017-0145104 A1 | 25 May 2017 |
| | | US | 2018-0002432 A1 | 04 January 2018 |
| | | US | 2020-0079865 A1 | 12 March 2020 |
| | | US | 2020-0115463 A1 | 16 April 2020 |
| | | US | 9228016 B2 | 05 January 2016 |
| | | US | 9745379 B2 | 29 August 2017 |
| | | UY | 36154 A | 08 January 2016 |
| | | WO | 2015-187835 A2 | 10 December 2015 |
| | | WO | 2015-187835 A3 | 28 January 2016 |

Form PCT/ISA/210 (patent family annex) (July 2019)

## REFERENCES CITED IN THE DESCRIPTION

**Patent documents cited in the description**

- KR 1020210060012

- WO 2015187835 A

**Non-patent literature cited in the description**

- **HEINE et al.** *Oncoimmunology,* 2017

- **SCHMIEDEL et al.** *Molecular Therapy,* 2013